# EUROPEAN PATENT APPLICATION

(11) **EP 4 471 064 A1**
(43) Date of publication of application: **04.12.2024**
(21) Application number: 23746362.5
(22) Date of filing: 28.01.2023
(51) Int. Cl.: C07K 16/28, C12N 5/10, C12N 15/13, A61K 39/395, A61P 35/00

(54) **ANTIGEN-BINDING PROTEIN AND USE THEREOF**

(30) Priority: 29.01.2022 CN 202210110741
(71) Applicant: Minghui Pharmaceutical (Hangzhou) Limited, Hangzhou, Zhejiang 310018 (CN); Minghui Pharmaceutical (Shanghai) Limited, Pilot Free Trade Zone Pudong New Area Shanghai 201203 (CN)
(72) Inventor: CAO, Guoqing, Hangzhou, Zhejiang 310018 (CN); ZHU, Liyuan, Hangzhou, Zhejiang 310018 (CN); SHI, Junwei, Hangzhou, Zhejiang 310018 (CN); LIU, Bo, Hangzhou, Zhejiang 310018 (CN)
(74) Representative: AWA Denmark A/S
(86) International application number: PCT/CN2023/073502
(87) International publication number: WO 2023/143484

(57) **Abstract**

The present application relates to an antigen-binding protein and use thereof, and specifically relates to an antigen-binding protein capable of inhibiting competitive binding of IGF-1R to ligands thereof with an IC₅₀ value of about 1.0 µg/mL or less in a competitive ELISA.

## Description

### TECHNICAL FIELD

The present application relates to the field of biomedicine, and in particular relates to an antigen-binding protein and use thereof.

### BACKGROUND ART

Interaction between IGF-1 (insulin-like growth factor I) and IGF-1R (insulin-like growth factor receptor 1) activates a cell signaling pathway by triggering autophosphorylation of tyrosine residues of the receptor. Activation of the receptor may stimulate growth and survival of tumor cells. Therefore, inhibiting IGF-1R activity is a potential approach to treat or prevent tumors. Overexpression of IGF-1R has also been found in several cancer cell lines and tumor tissues, and the degree of IGF-1R expression may be correlated with disease severity. There is an urgent need in the art for a method capable of effectively inhibiting the activity of IGF-1R, for example, an antigen-binding protein or a functionally active fragment thereof that may bind to IGF-1R.

### SUMMARY

The present application provides an antigen-binding protein, which may have properties selected from a group consisting of: (1) inhibiting IGF-1R from binding to ligands thereof; (2) binding to IGF-1R; (3) selectively binding to primate (e.g., human or monkey) IGF-1R; (4) inhibiting proliferation of cells overexpressing IGF-1R; (5) inhibiting proliferation of human breast cancer cells, e.g., inhibiting IGF-1-induced proliferation of human breast cancer cells; and (6) inhibiting IGF-1-induced MEK, Erk1/2 and/or Akt phosphorylation.

In one aspect, the present application provides an antigen-binding protein, capable of inhibiting competitive binding of IGF-1R to ligands thereof with an IC50 value of about 1.0 µg/mL or less in a competitive ELISA.

In one aspect, the present application provides an antigen-binding protein, capable of competing with a reference antibody to bind to IGF-1R, wherein the reference antibody includes HCDR1 to 3, and the HCDR1 to 3 include the amino acid sequences set forth in SEQ ID NOs: 85, 100 and 131, respectively.

In one aspect, the present application provides an antigen-binding protein, including HCDR3, wherein the HCDR3 includes the amino acid sequence set forth in SEQ ID NO: 217 or 218.

In one aspect, the present application provides a polypeptide, including the antigen-binding protein described herein.

In one aspect, the present application provides an immunoconjugate, including the isolated antigen-binding protein described herein and/or the polypeptide described herein.

In one aspect, the present application provides a nucleic acid, encoding the isolated antigen-binding protein described herein and/or the polypeptide described herein.

In one aspect, the present application provides a vector, including the nucleic acid described herein.

In one aspect, the present application provides a cell, including the isolated antigen-binding protein described herein, the polypeptide described herein, the immunoconjugate described herein, the nucleic acid described herein, and/or the vector described herein.

In one aspect, the present application provides a method for preparing the isolated antigen-binding protein described herein and/or the polypeptide described herein, wherein the method includes culturing the cell described herein under the conditions of allowing expression of the isolated antigen-binding protein and/or the polypeptide.

In one aspect, the present application provides a composition, including the isolated antigen-binding protein described herein, the polypeptide described herein, the immunoconjugate described herein, the nucleic acid described herein, the vector described herein, and/or the cell described herein, and optionally a pharmaceutically acceptable adjuvant.

In one aspect, the present application provides a kit, including the isolated antigen-binding protein described herein, the polypeptide described herein, the immunoconjugate described herein, the nucleic acid described herein, the vector described herein, the cell described herein, and/or the composition described herein.

In one aspect, the present application provides a method for inhibiting proliferation of cells overexpressing IGF-1R or a functionally active fragment thereof, inhibiting an MAPK pathway and/or inhibiting interaction between IGF-1R or a functionally active fragment thereof and ligands thereof, including administering the isolated antigen-binding protein described herein, the polypeptide described herein, the immunoconjugate described herein, the nucleic acid described herein, the vector described herein, the cell described herein, the composition described herein, and/or the kit described herein.

In one aspect, the present application provides a method for detecting the presence and/or content of IGF-1R or a functionally active fragment thereof, including administering the isolated antigen-binding protein described herein, the polypeptide described herein, the immunoconjugate described herein, the nucleic acid described herein, the vector described herein, the cell described herein, the composition described herein, and/or the kit described herein.

In one aspect, the present application provides use of the isolated antigen-binding protein described herein, the polypeptide described herein, the immunoconjugate described herein, the nucleic acid described herein, the vector described herein, the cell described herein, the composition described herein, and/or the kit described herein in preparation of a drug for preventing, alleviating, and/or treating diseases or disorders.

Those skilled in the art can easily discern other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
FIG. 1 shows an SDS-PAGE diagram of a nanobody described herein.
FIG. 2 shows the results of a nanobody-Fc fusion protein described herein binding to recombinant human, monkey and mouse IGF 1R proteins and a human insulin receptor protein.
FIG. 3 shows the results of the inhibitory effect of the nanobody-Fc fusion protein described herein on IGF-1-induced Erk protein phosphorylation.
FIG. 4 shows the inhibitory effect of the nanobody-Fc fusion protein described herein on IGF-1-induced MEK, Erk and Akt protein phosphorylation (note: Fc' indicates a subtype of IgG1 C220S L234A L235A D356E L358M).

### DETAILED DESCRIPTION OF THE INVENTION

The embodiments of the invention of the present application will be described below with specific examples. Those skilled in the art can easily understand other advantages and effects of the invention of the present application from the disclosure of the specification.

### Definition of Terms

In the present application, the term "antigen-binding protein" generally refers to a polypeptide molecule capable of specifically recognizing and/or neutralizing a specific antigen. In the present application, the term "antigen-binding protein" may include an "antibody" or an "antigen-binding fragment". The term "antibody" may include a monoclonal antibody, an antibody fragment, or an antibody derivative, including, but not limited to, an alpaca antibody, an all-human antibody, a humanized antibody, a chimeric antibody, a single chain antibody (e.g., scFv), and antibody fragments (e.g., Fab, Fab' and (Fab)2 fragments) that bind to an antigen. The term "antibody" may also include all recombinant forms of an antibody, such as an antibody expressed in prokaryotic cells, an unglycosylated antibody, and any antigen-binding antibody fragment and derivative thereof as described herein. In the present application, the "antibody" may include a single domain antibody.

In the present application, the term "isolated" generally refers to those obtained from a natural state by manual means. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude substances mixed with artificial or synthetic substances, nor does it exclude the existence of other impurities that do not affect the activity of the substance.

In the present application, the term "single domain antibody" generally refers to a class of antibodies that have a light chain deletion of the antibody and only a variable domain of the heavy chain. Studies have found that there is a heavy chain antibody (hcAb) including only heavy chains but with complete functions in two-humped camels, one-humped camels, alpacas and llamas. The molecular weight of a variable domain of the hcAb (VHH) is only 1/10 of that of a conventional antibody. The heavy chain antibody is the smallest molecular fragment with complete antibody functions that may be obtained at present, and is called a single domain antibody (sdAb). Compared with other antibodies, the single domain antibody has the advantages of low immunogenicity, small molecules, strong penetration, etc., and thus has broad application prospects in basic research, drug development, disease treatment and other fields. For example, a single domain antibody may be derived from an alpaca. A single domain antibody may include a variable domain of the heavy chain (VH). The term "variable domain of the heavy chain" usually refers to the amino terminal domain of the heavy chain of an antigen-binding fragment. A variable domain of the heavy chain may be further divided into hypervariable regions termed complementarity determining regions (CDRs), interspersed in more conserved regions called framework regions (FRs). Each variable domain of the heavy chain may include three CDRs and four FR regions, which may be arranged in the following order from an amino terminus to a carboxyl terminus: H-FR1, HCDR1, H-FR2, HCDR2, H-FR3, HCDR3 and H-FR4. A variable domain of the heavy chain includes a binding domain that interacts with the antigen described herein. The exact boundaries of the CDRs have been defined differently according to different systems. A system described by Kabat not only provides an unambiguous residue numbering system applicable to any variable region of an antigen-binding protein, but also provides precise residue boundaries that define the CDRs. These CDRs may be referred to as Kabat CDRs. Chothia and colleagues found that despite the large diversity at the amino acid sequence level, certain subunits within the Kabat CDRs adopt nearly identical peptide backbone conformations. These subunits are named L1, L2, and L3 or H1, H2, and H3, wherein "L" and "H" refer to light and heavy chain regions, respectively. These regions may be referred to as Chothia CDRs, and the Chothia CDRs have boundaries that overlap with the Kabat CDRs. Other boundaries defining the CDRs that overlap with the Kabat CDRs have been described by Padlan and MacCallum. In addition, other CDR boundary definitions may not strictly follow one of the above systems but will still overlap with the Kabat CDRs, although they may be shortened or lengthened, found by a prediction or experiment in which specific residues, or groups of residues, or even the entire CDRs, do not significantly affect antigen binding. In the present application, the CDRs may be defined using the Contact numbering system. In the present application, the CDRs may be defined using the Abm numbering system. In the present application, the CDRs may be defined using the Chothia numbering system. In the present application, the CDRs may be defined using the Kabat numbering system. In the present application, the CDRs may be defined using the IMGT numbering system. In the present application, the term "single domain antibody" is used interchangeably with "nanobody" and "VHH". Although the scope of protection set forth in the present application includes the sequences shown based on definition of the IMGT numbering system, the amino acid sequences corresponding to other definition rules of CDRs should also fall within the scope of protection of the present application. Exemplary definition rules are shown in a table below.

In the present application, the term "monoclonal antibody" generally means a group of substantially homologous antibodies, i.e., the antibodies in the group are identical except for possible naturally occurring mutations in trace presence. A monoclonal antibody is highly specific, and targets a single antigenic site directly. For example, the monoclonal antibody may be prepared using hybridoma technology or produced in bacterial, eukaryotic, or plant cells by a recombinant DNA method. The monoclonal antibody may also be obtained from a phage antibody library using technologies known in the art.

In the present application, the term "chimeric antibody" generally refers to an antibody in which a moiety of each heavy- or light-chain amino acid sequence is homologous to, or belongs to a particular class of, a corresponding amino acid sequence of an antibody from a particular species, and the remaining segments of the chain are homologous to a corresponding sequence in another species. For example, the variable regions of the light and heavy chains are derived from the variable regions of antibodies from one animal species (such as mouse and rat), while the constant moieties are homologous to antibody sequences from another species (such as human). For example, to obtain a chimeric antibody, the variable regions may be produced using non-human B cells or hybridoma cells, and a constant region combined therewith is from a human. The variable regions have the advantage of being easy to prepare, and the specificity thereof is not affected by the origin of a constant region combined therewith. Also, since the constant region of a chimeric antibody may be derived from human, the possibility of the chimeric antibody triggering immune response when injected is lower than that of antibodies with a constant region of a non-human origin.

In the present application, the term "IGF-1R", "IGF1R", "IGF-IR", or "IGFIR" generally refers to insulin-like growth factor receptor 1. The IGF-1R described herein may include equivalents thereof, species homologs, functionally active fragments, and analogs with at least one epitope in common with the IGF-1R. For example, the "functionally active fragments" may include fragments that retain the endogenous function of at least one naturally occurring protein. An exemplary amino acid sequence of IGF-1R may be shown in UniProt Accession No. P08069. For example, the ligands of IGF-1R may include IGF-1 and/or IGF-2.

In the present application, the term "IGF-1", "IGF1", "IGF-I", or "IGFI" generally refers to insulin-like growth factor 1. The IGF-1 described herein may include equivalents thereof, species homologs, functionally active fragments, and analogs with at least one epitope in common with the IGF-1. For example, the "functionally active fragments" may include fragments that retain the endogenous function of at least one naturally occurring protein. An exemplary amino acid sequence of IGF-1 may be shown in UniProt Accession No. P05019.

In the present application, the term "IGF-2", "IGF2", "IGF-II", or "IGFII" generally refers to insulin-like growth factor 2. The IGF-2 described herein may include equivalents thereof, species homologs, functionally active fragments, and analogs with at least one epitope in common with the IGF-2. For example, the "functionally active fragments" may include fragments that retain the endogenous function of at least one naturally occurring protein. An exemplary amino acid sequence of IGF-2 may be shown in UniProt Accession No. P01344.

In addition to specific proteins and nucleotides mentioned herein, the present application may also include functional variants, derivatives, analogs, homologs and fragments thereof.

The term "functional variant" refers to a polypeptide having substantially the same amino acid sequence as a naturally occurring sequence or encoded by substantially the same nucleotide sequence, and having one or more activities of the naturally occurring sequence. In the context of the present application, a variant of any given sequence refers to a sequence in which a specific sequence of a residue (whether an amino acid or nucleotide residues) has been modified such that the polypeptide or polynucleotide substantially retains at least one endogenous function. Variant sequences may be obtained by addition, deletion, substitution, modification, replacement and/or variation of at least one amino acid residue and/or nucleotide residue present in a naturally occurring protein and/or polynucleotide, provided that the original functional activity is maintained.

In the present application, the term "derivative" generally refers to a polypeptide or polynucleotide described herein including any substitution, variation, modification, replacement, deletion and/or addition of one (or more) amino acid residue(s) from/to a sequence, provided that the resulting polypeptide or polynucleotide substantially retains at least one of the endogenous functions thereof.

In the present application, the term "analog" generally refers to a polypeptide or polynucleotide, including any mimetic of the polypeptide or polynucleotide, i.e., a chemical compound having at least one endogenous function of the polypeptide or polynucleotide simulated by the mimetic.

Generally, amino acid substitutions, e.g., at least 1 (e.g., 1, 2, 3, 4, 5, 6, 7, 8, 9, 10 or 20 or more) amino acid substitution, may be conducted provided that the modified sequence substantially retains the desired activity or ability. Amino acid substitutions may include use of non-naturally occurring analogs.

In the present application, the term "homolog" generally refers to an amino acid sequence or nucleotide sequence that has a certain homology to a naturally occurring sequence. The term "homology" may be equated with sequence "identity". Homologous sequences may include amino acid sequences that may be at least 80%, 85%, 90%, 99.1%, 99.2%, 99.3%, 99.4%, 99.5%, 99.6%, 99.7%, 99.8%, or 99.9% identical to a subject sequence. Generally, homologs will include the same active sites etc. as a subject amino acid sequence. Homology may be considered in terms of similarity (i.e., amino acid residues having similar chemical properties/functions), or may be expressed in terms of sequence identity. In the present application, a sequence having a percentage identity to any one of the SEQ ID NOs. of an amino acid sequence or nucleotide sequence mentioned refers to a sequence having the said percentage identity over the entire length of the SEQ ID NO mentioned. To determine the sequence identity, sequence alignment may be conducted by various means known to those skilled in the art, for example, BLAST, BLAST-2, ALIGN, NEEDLE or Megalign (DNASTAR) software. Those skilled in the art can determine appropriate parameters for alignment, including any algorithms required to achieve optimal alignment across the full-length sequences being compared.

A protein or a polypeptide used herein may also have deletion, insertion or substitution of an amino acid residue which produces silent changes and result in a functionally equivalent protein. Deliberate amino acid substitutions may be conducted on the basis of similarity in polarity, charge, solubility, hydrophobicity, hydrophilicity, and/or the amphipathic nature of residues, provided that endogenous functions are retained. For example, negatively charged amino acids include aspartic acid and glutamic acid; positively charged amino acids include lysine and arginine; and amino acids including uncharged polar head groups with similar hydrophilicity values include asparagine, glutamine, serine, threonine, and tyrosine.

In the present application, the term "tumor" generally refers to a neoplasm that results from proliferation of local tissue cells under the action of various tumorigenic factors. For example, the tumor may include a solid tumor. For example, the tumor may include a blood tumor. For example, the tumor may include a tumor associated with expression of an antigen described herein. The term "tumor associated with expression of an antigen described herein" generally refers to a tumor that results from a change in expression of the antigen described herein resulting in progression of a disease or evasion of immunological surveillance. For example, the "tumor associated with expression of an antigen described herein" may refer to a tumor that results from up-regulation in expression of the antigen described herein resulting in progression of a disease or evasion of immunological surveillance. The tumor associated with protein expression of an antigen described herein may refer to a tumor positive for the antigen described herein. In a tumor that is positive for the antigen described herein, the protein expression of the antigen described herein on the surface of tumor cells or in a tumor microenvironment is about 1%, 5%, 10%, 15%, 20%, 25%, 30%, 35%, 40%, 50%, 60%, 70%, 80% or higher than that in normal cells.

In the present application, the term "solid tumor" generally refers to a concrete lump that may be detected by clinical examination, for example, X-ray, CT scan, B-mode ultrasonography, or palpation. For example, the solid tumor may be selected from breast cancer.

In the present application, the term "blood tumor" generally refers to a tumor that cannot be seen or palpated by X ray, CT scan, B-mode ultrasonography, and palpation.

In the present application, the term "immunoconjugate" generally refers to a conjugate formed by conjugation of another agent (e.g., a chemotherapeutic agent, a radioactive element, a cytostatic agent, and a cytotoxic agent) to the isolated antigen-binding protein (e.g., covalently linked by a linker molecule), which may deliver the other agent to a target cell (e.g., a tumor cell) by the isolated antigen-binding protein specifically binding to an antigen on the target cell. The immunoconjugate is then internalized and eventually enters the interior of the target cell (e.g., into a vesicle such as a lysosome), at which point the linker molecule in the immunoconjugate may cleave to release the other agent to exert the cytotoxic effect. In addition, the antigen may also be secreted by the target cell and located in the space outside the target cell.

In the present application, the term "subject" generally refers to a human or a non-human animal, including but not limited to a cat, a dog, a horse, a pig, a cow, a sheep, a rabbit, a mouse, a rat, or a monkey.

In the present application, the term "nucleic acid molecule" generally refers to an isolated form of a nucleotide, a deoxyribonucleotide or a ribonucleotide or an analog thereof of any length that is isolated from their natural environment or artificially synthesized.

In the present application, the term "vector" generally refers to a nucleic acid molecule capable of transferring another nucleic acid linked thereto. The vector may transfer the inserted nucleic acid molecule into cells and/or between cells. The vector may include a vector primarily used for insertion of DNA or RNA into cells, a vector primarily used for replication of DNA or RNA, and a vector primarily used for expression of transcription and/or translation of DNA or RNA. The vector may be a polynucleotide capable of being transcribed and translated into a polypeptide when introduced into a suitable cell. Generally, the vector may produce the desired expression product by culturing appropriate cells including the vector. In the present application, the vector may include a lentiviral vector.

In the present application, the term "cell" generally refers to an individual cell, a cell line or a cell culture which may include or have included a plasmid or a vector including the nucleic acid molecule described herein, or which is capable of expressing the polypeptide described herein or the antigen-binding protein described herein. The cell may include a progeny of a single cell. Due to natural, accidental or intentional mutations, a progeny cell may not necessarily be completely identical in morphology or genome to the original parent cell, provided that the progeny cell is capable of expressing the polypeptide or the antigen-binding protein described herein. The cell may be obtained by transfecting a cell in vitro using the vector described herein. The cell may be a prokaryotic cell (e.g., E. coli) or an eukaryotic cell (e.g., a yeast cell, such as a COS cell, a Chinese hamster ovary (CHO) cell, a HeLa cell, a HEK293 cell, a COS-1 cell, an NS0 cell or a myeloma cell). In some embodiments, the cell may be an immune cell. For example, the immune cell may be selected from the group consisting of a T cell, a B cell, a natural killer cell (NK cell), a macrophage, an NKT cell, a monocyte, a dendritic cell, a granulocyte, a lymphocyte, a leukocyte and/or a peripheral blood mononuclear cell.

In the present application, the term "treatment" generally refers to: (i) preventing the emergence of diseases, disorders and/or conditions of illness in a patient who may be prone to the diseases, disorders and/or conditions of illness but has not yet been diagnosed; (ii) inhibiting the diseases, disorders or conditions of illness, that is, controlling the development thereof; and (iii) alleviating the diseases, disorders or conditions of illness, that is, causing the diseases, disorders and/or conditions of illness and/or the symptoms associated with the diseases, disorders and/or conditions of illness to subside.

In the present application, the terms "polypeptide", "peptide", and "protein" are used interchangeably, and generally refer to polymers of amino acids of any length. The polymers may be linear or branched, may include modified amino acids, and may be interrupted by non-amino acids. The terms also encompass amino acid polymers that have been modified. The modifications may include: disulfide bond formation, glycosylation, lipidation, acetylation, phosphorylation, or any other manipulation (e.g., conjugation to a labeling moiety). The term "amino acids" include natural and/or non-natural or synthetic amino acids, including glycine and D and L optical isomers, as well as amino acid analogs and peptide mimics.

In the present application, the terms "polynucleotide", "nucleotide", "nucleotide sequence", "nucleic acid" and "oligonucleotide" are used interchangeably, and generally refer to polymeric forms of nucleotides of any length, such as deoxyribonucleotides or ribonucleotides, or analogs thereof. A polynucleotide may have any three-dimensional structure and may perform any function, known or unknown. The following are non-limiting examples of polynucleotides: coding or non-coding regions of a gene or gene fragment, multiple loci (a locus) defined by linkage analysis, exons, introns, messenger RNA (mRNA), transfer RNA, ribosomal RNA, short interfering RNA (siRNA), short hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. Polynucleotides may include one or more modified nucleotides, such as methylated nucleotides and nucleotide analogs. If present, modifications to the nucleotide structure may be conducted before or after polymer assembly. The sequence of nucleotides may be interrupted by non-nucleotide components. Polynucleotides may be further modified after polymerization, for example, by conjugation with a labeling moiety.

In the present application, the term "K_{D}" (similarly, "*K_{D}*" or "KD") generally refers to the "affinity constant" or "equilibrium dissociation constant", and to a value obtained at equilibrium in a titration measurement or by dividing the dissociation rate constant (k_{d}) by the binding rate constant (kₐ). The binding affinity of a binding protein (e.g., the isolated antigen-binding protein described herein) for an antigen (e.g., the antigen protein described herein) is expressed using the association rate constant (kₐ), the dissociation rate constant (k_{d}), and the equilibrium dissociation constant (K_{D}). Methods for determining the association and dissociation rate constants are well known in the art. The use of fluorescence-based technology provides high sensitivity and the ability to examine samples at equilibrium in a physiological buffer. For example, the *K_{D}* value may be determined by an Octet Molecular Interaction Analyzer (Sartorius), or by using other experimental approaches and instruments such as a BIAcore Biomolecular Interaction Analysis System (GE Healthcare Life Sciences). In addition, the *K_{D}* value may also be determined using a KinExA system (Kinetic Exclusion Assay) from Sapidyne Instruments, or the *K_{D}* value may be determined using other surface plasmon resonance (SPR) instruments. In the present application, the *K_{D}* value may be determined by BIAcore.

In the present application, the term "and/or" shall be understood to mean either one or both of the options.

In the present application, the term "including" generally refers to inclusion of clearly specified features, but does not exclude other elements. In some cases, "including" also encompasses the situation where only the specified components are included. In some cases, "including" also means "consisting of'.

In the present application, the term "about" generally refers to a change in a range of 0.5-10% greater or less than a specified value, for example, a change in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater or less than a specified value.

### DETAILED DESCRIPTION OF THE INVENTION

The present application provides an antigen-binding protein, capable of inhibiting competitive binding of IGF-1R to ligands thereof with an IC50 value of about 1.0 µg/mL or less in a competitive ELISA. The isolated antigen-binding protein described herein may inhibit IGF-1 from binding to IGF-1R with an EC₅₀ of about 2 µg/mL or less (e.g., the EC₅₀ is not higher than about 1.9 µg/mL, not higher than about 1.8 µg/mL, not higher than about 1.7 µg/mL, not higher than about 1.5 µg/mL, not higher than about 1.3 µg/mL, not higher than about 1.1 µg/mL, not higher than about 1.0 µg/mL, not higher than about 0.9 µg/mL, not higher than 0.8 µg/mL, not higher than about 0.7 µg/mL, not higher than 0.6 µg/mL, not higher than about 0.5 µg/mL, not higher than about 0.4 µg/mL, not higher than about 0.3 µg/mL, not higher than about 0.2 µg/mL, or not higher than about 0.1 µg/mL or less).

For example, the IGF-1R may include IGF-1R derived from a primate. For example, the ligands of IGF-1R may include IGF-1 and/or IGF-2, and functionally active fragments thereof.

The present application provides an antigen-binding protein, the antigen-binding protein may compete with a reference antibody to bind to IGF-1R, the reference antibody may include HCDR1 to 3, and the HCDR1 to 3 may include the amino acid sequences set forth in SEQ ID NOs: 85, 100 and 131, respectively. In the present application, the CDRs may be defined using the Contact numbering system. In the present application, the CDRs may be defined using the Abm numbering system. In the present application, the CDRs may be defined using the Chothia numbering system. In the present application, the CDRs may be defined using the Kabat numbering system. In the present application, the CDRs may be defined using the IMGT numbering system. Those skilled in the art may determine CDR regions using different coding systems based on the sequence and structure of an antibody. Using different encoding systems, the CDR regions may differ. In the present application, the CDR encompasses CDR sequences divided according to any CDR division method; the CDR also encompasses variants thereof including amino acid sequences of the CDR after substitution, deletion and/or addition of one or more amino acids, for example, substitution, deletion and/or insertion of 1-30, 1-20 or 1-10, or 1, 2, 3, 4, 5, 6, 7, 8 or 9 amino acids; and the CDR also encompasses homologs thereof which may be amino acid sequences having at least about 85% (e.g., at least about 85%, about 90%, about 91%, about 92%, about 93%, about 94%, about 95%, about 96%, about 97%, about 98%, about 99% or higher) sequence homology with the amino acid sequence of the CDR. In certain embodiments, the CDR may be determined by the Kabat numbering scheme.

For example, the reference antibody may include a variable domain of the heavy chain (VH), and the VH may include the amino acid sequence set forth in SEQ ID NO: 1. For example, the reference antibody may be the antibody 5115 described herein.

In the present application, the antigen-binding protein may include a variable domain of the heavy chain VH, and the VH may include at least one, two or three of HCDR1, HCDR2 and HCDR3. For example, the sequence of HCDR3 of the antigen-binding protein may be determined according to the Kabat definition rules.

For example, the present application provides multiple CDR definition methods. Taking the antibody 5115 as an example:

| | HCDR1 (SEQ ID NO) | HCDR2 (SEQ ID NO) | HCDR3 (SEQ ID NO) |
|---|---|---|---|
| Contact | SSFVMG (8 1) | FVAAISGSGSRAN (96) | AANPRRATPDYTQYA (130) |
| Abm | GRTFSSFVMG (82) | AISGSGSRAN (97) | NPRRATPDYTQYAY (131) |
| Chothia | GRTFSSF (83) | SGSGSR (98) | NPRRATPDYTQYAY (131) |
| IMGT | GRTFSSFV (84) | ISGSGSRA (99) | AANPRRATPDYTQYAY (132) |
| Kabat | SFVMG (85) | AISGSGSRANYADSVKG (100) | NPRRATPDYTQYAY (131) |

The present application provides an antigen-binding protein, which may include HCDR3, and the HCDR3 may include the amino acid sequence set forth in SEQ ID NO: 217 (NPR[RY]AT[PT][DG][FLMY][HIKLMQRST][AQRS]Y[AKL]Y) or 218 (NPRRATPD[LY]TQY[AL]Y). The letters in square brackets indicate the amino acid that can be selected at that site.

For example, the antigen-binding protein may include HCDR3, and the HCDR3 may include the amino acid sequence set forth in any one of SEQ ID NOs: 130-153. The CDRs may be defined using the Kabat numbering system.

For example, the antigen-binding protein may include HCDR2, and the HCDR2 may include the amino acid sequence set forth in SEQ ID NO: 215 (AISG[FGHKQRST]G[IKLMQRST][QRV][AFHLMQSTVY][LNRTY]YADSVKG) or 216 (AISGSG[MS]RA[RY]YADSVKG). The letters in square brackets indicate the amino acid that can be selected at that site.

For example, the antigen-binding protein may include HCDR2, and the HCDR2 may include the amino acid sequence set forth in any one of SEQ ID NOs: 96-129. The CDRs may be defined using the Kabat numbering system.

For example, the antigen-binding protein may include HCDR1, and the HCDR1 may include the amino acid sequence set forth in SEQ ID NO: 213 ([GRST][FLY]V[LM][AGMS]) or 214 (S[FL]V[LM][AS]). The letters in square brackets indicate the amino acid that can be selected at that site.

For example, the antigen-binding protein may include HCDR1, and the HCDR1 may include the amino acid sequence set forth in any one of SEQ ID NOs: 81-95. The CDRs may be defined using the Kabat numbering system.

For example, the antigen-binding protein may include a variable domain of the heavy chain (VH); the VH may include HCDR1, HCDR2 and HCDR3; the HCDR3 may include the amino acid sequence set forth in SEQ ID NO: 217 or 218; the HCDR2 may include the amino acid sequence set forth in SEQ ID NO: 215 or 216; and the HCDR1 may include the amino acid sequence set forth in SEQ ID NO: 213 or 214.

For example, the antigen-binding protein may include a variable domain of the heavy chain (VH); the VH may include the HCDR1, HCDR2 and HCDR3; the HCDR3 may include the amino acid sequence set forth in any one of SEQ ID NOs: 130-153; the HCDR2 may include the amino acid sequence set forth in any one of SEQ ID NOs: 96-129; and the HCDR1 may include the amino acid sequence set forth in any one of SEQ ID NOs: 81-95.

For example, the antigen-binding protein may include H-FR1, the C-terminus of the H-FR1 is directly or indirectly linked to the N-terminus of the HCDR1, and the H-FR1 may include the amino acid sequence set forth in any one of SEQ ID NOs: 219 (EVQLVES [GS] GGLVQ [AP] G[DG] SLRL SCA[AV] S [GHLMRST] [AGIKPRTV] [AEFGPRST Y][FLR][KNPRS]), 220 (EVQLVESGGGLVQPGGSLRLSCAASGRTF[RS]), and 154-186. The letters in square brackets indicate the type of an amino acid that may be selected at that site.

For example, the antigen-binding protein may include H-FR2, the H-FR2 is located between the HCDR1 and the HCDR2, and the H-FR2 may include the amino acid sequence set forth in any one of SEQ ID NOs: 221 (WFRQAPGK[EG][LR]E[FL]V[AS]), 222 (WFRQAPGK[EG][LR]EFVS), and 187-195. The letters in square brackets indicate the type of an amino acid that may be selected at that site.

For example, the antigen-binding protein may include H-FR3, the H-FR3 is located between the HCDR2 and the HCDR3, and the H-FR3 may include the amino acid sequence set forth in any one of SEQ ID NOs: 223 (RF [AT] [IV] SRDN[AS] [KN]NT[LV]YLQM[NS] SL[KR] [AP] [DE]DT[AG] [LV]YYCAA) and 196-203. The letters in square brackets indicate the type of an amino acid that may be selected at that site.

For example, the antigen-binding protein may include H-FR4, the N-terminus of the H-FR4 is directly or indirectly linked to the C-terminus of the HCDR3, and the H-FR4 may include the amino acid sequence set forth in any one of SEQ ID NOs: 224 (WGQGT[LQ]VTVSS) and 204-206. The letters in square brackets indicate the type of an amino acid that may be selected at that site.

For example, the antigen-binding protein may include H-FR1, H-FR2, H-FR3 and H-FR4; the H-FR1 may include the amino acid sequence set forth in SEQ ID NO: 219 or 220; the H-FR2 may include the amino acid sequence set forth in SEQ ID NO: 221 or 222; the H-FR3 may include the amino acid sequence set forth in SEQ ID NO: 223; and the H-FR4 may include the amino acid sequence set forth in SEQ ID NO: 224.

For example, the amino acid information (SEQ ID NO) of the respective regions corresponding to the Nos. of antigen-binding proteins described herein may be as follows:

| No. of antigen -binding protein | HCDR1 | HCDR2 | HCDR3 | FR1 | FR2 | FR3 | FR4 |
|---|---|---|---|---|---|---|---|
| 5115 | 85 | 100 | 131 | 156 | 188 | 200 | 205 |
| 5666 | 86 | 101 | 133 | 156 | 191 | 201 | 205 |
| 5115-H0 | 87 | 102 | 131 | 157 | 192 | 202 | 205 |
| 5115-H1 | 87 | 102 | 131 | 157 | 192 | 202 | 206 |
| 5115-H2 | 87 | 102 | 131 | 157 | 193 | 202 | 206 |
| 5115-H3 | 87 | 102 | 131 | 157 | 194 | 202 | 206 |
| 5115-H4 | 87 | 102 | 131 | 157 | 195 | 202 | 206 |
| 5115-H5 | 88 | 102 | 131 | 157 | 195 | 202 | 206 |
| 5115-H6 | 88 | 102 | 131 | 157 | 192 | 202 | 206 |
| 5115-H4-M3 | 89 | 103 | 131 | 157 | 195 | 202 | 206 |
| 5115-H4-M10 | 87 | 104 | 134 | 158 | 195 | 202 | 206 |
| 5115-H4-M12 | 87 | 105 | 134 | 157 | 195 | 202 | 206 |
| 5115-H4-M24 | 90 | 103 | 134 | 159 | 195 | 202 | 206 |
| 5115-H4-M25 | 87 | 106 | 134 | 160 | 195 | 202 | 206 |
| 5115-H4-M28 | 87 | 107 | 135 | 157 | 195 | 203 | 206 |
| 5115-H4-M34 | 89 | 103 | 134 | 157 | 195 | 202 | 206 |
| 5115-H4-M39 | 87 | 108 | 135 | 157 | 195 | 202 | 206 |
| 5115-H4-M41 | 85 | 103 | 136 | 157 | 195 | 202 | 206 |
| 5115-H4-M44 | 87 | 103 | 134 | 161 | 195 | 202 | 206 |
| 5115-H4-M48 | 90 | 102 | 135 | 162 | 195 | 202 | 206 |
| 5115-H4-M52 | 87 | 100 | 134 | 163 | 195 | 202 | 206 |
| 5115-H4-M59 | 87 | 109 | 137 | 164 | 195 | 202 | 206 |
| 5115-H4-M61 | 91 | 103 | 134 | 157 | 195 | 202 | 206 |
| 5115-H4-M69 | 87 | 103 | 134 | 165 | 195 | 202 | 206 |
| 5115-H1-M1 | 87 | 108 | 131 | 157 | 192 | 202 | 206 |
| 5115-H1-M2 | 87 | 103 | 138 | 157 | 192 | 202 | 206 |
| 5115-H1-M3 | 87 | 103 | 134 | 157 | 192 | 202 | 206 |
| 5115-H1-M4 | 87 | 110 | 131 | 166 | 192 | 202 | 206 |
| 5115-H1-M5 | 89 | 111 | 139 | 167 | 195 | 202 | 206 |
| 5115-H1-M6 | 89 | 103 | 139 | 168 | 195 | 202 | 206 |
| 5115-H1-M7 | 87 | 109 | 135 | 157 | 192 | 202 | 206 |
| 5115-H1-M8 | 87 | 112 | 140 | 157 | 192 | 202 | 206 |
| 5115-H1-M9 | 87 | 113 | 138 | 157 | 192 | 202 | 206 |
| 5115-H1-M10 | 87 | 102 | 141 | 169 | 192 | 202 | 206 |
| 5115-H1-M11 | 87 | 114 | 131 | 170 | 192 | 202 | 206 |
| 5115-H1-M12 | 87 | 103 | 135 | 157 | 192 | 202 | 206 |
| 5115-H1-M13 | 87 | 103 | 131 | 171 | 192 | 202 | 206 |
| 5115-H1-M14 | 87 | 115 | 134 | 157 | 192 | 202 | 206 |
| 5115-H1-M15 | 87 | 115 | 135 | 157 | 192 | 202 | 206 |
| 5115-H1-M16 | 87 | 103 | 134 | 172 | 192 | 202 | 206 |
| 5115-H1-M17 | 87 | 108 | 142 | 157 | 192 | 202 | 206 |
| 5115-H1-M18 | 87 | 103 | 134 | 161 | 192 | 202 | 206 |
| 5115-H1-M19 | 87 | 115 | 143 | 157 | 192 | 202 | 206 |
| 5115-H1-M20 | 87 | 116 | 131 | 165 | 192 | 202 | 206 |
| 5115-H1-M21 | 87 | 117 | 131 | 173 | 192 | 202 | 206 |
| 5115-H1-M22 | 87 | 108 | 138 | 157 | 192 | 202 | 206 |
| 5115-H1-M23 | 87 | 103 | 137 | 174 | 192 | 202 | 206 |
| 5115-H1-M24 | 92 | 118 | 134 | 157 | 195 | 202 | 206 |
| 5115-H1-M25 | 87 | 103 | 144 | 166 | 192 | 202 | 206 |
| 5115-H1-M26 | 87 | 119 | 145 | 157 | 192 | 202 | 206 |
| 5115-H1-M27 | 89 | 111 | 146 | 175 | 195 | 202 | 206 |
| 5115-H1-M28 | 87 | 116 | 147 | 157 | 192 | 202 | 206 |
| 5115-H1-M29 | 87 | 102 | 135 | 157 | 192 | 202 | 206 |
| 5115-H1-M30 | 87 | 114 | 131 | 176 | 192 | 202 | 206 |
| 5115-H1-M31 | 87 | 103 | 148 | 157 | 192 | 202 | 206 |
| 5115-H1-M32 | 87 | 120 | 131 | 177 | 192 | 202 | 206 |
| 5115-H1-M33 | 87 | 108 | 131 | 178 | 192 | 202 | 206 |
| 5115-H1-M34 | 87 | 115 | 148 | 157 | 192 | 202 | 206 |
| 5115-H1-M35 | 87 | 106 | 135 | 157 | 192 | 202 | 206 |
| 5115-H1-M36 | 87 | 102 | 135 | 179 | 192 | 202 | 206 |
| 5115-H1-M37 | 89 | 121 | 134 | 157 | 195 | 202 | 206 |
| 5115-H1-M38 | 89 | 121 | 138 | 168 | 195 | 202 | 206 |
| 5115-H1-M39 | 87 | 110 | 149 | 157 | 192 | 202 | 206 |
| 5115-H1-M40 | 87 | 112 | 135 | 157 | 192 | 202 | 206 |
| 5115-H4-M34-1 | 93 | 122 | 134 | 180 | 195 | 202 | 206 |
| 5115-H4-M34-2 | 89 | 123 | 146 | 181 | 195 | 202 | 206 |
| 5115-H4-M34-3 | 89 | 103 | 150 | 169 | 195 | 202 | 206 |
| 5115-H4-M34-4 | 89 | 124 | 134 | 169 | 195 | 202 | 206 |
| 5115-H4-M34-5 | 89 | 125 | 134 | 182 | 195 | 202 | 206 |
| 5115-H4-M34-6 | 89 | 108 | 146 | 157 | 195 | 202 | 206 |
| 5115-H4-M34-7 | 89 | 126 | 151 | 157 | 195 | 202 | 206 |
| 5115-H4-M34-8 | 94 | 108 | 134 | 157 | 195 | 202 | 206 |
| 5115-H4-M34-9 | 89 | 103 | 134 | 183 | 195 | 202 | 206 |
| 5115-H4-M34-10 | 89 | 127 | 152 | 163 | 195 | 202 | 206 |
| 5115-H4-M34-11 | 89 | 103 | 134 | 184 | 195 | 202 | 206 |
| 5115-H4-M34-12 | 89 | 103 | 146 | 185 | 195 | 202 | 206 |
| 5115-H4-M34-13 | 95 | 128 | 153 | 157 | 195 | 202 | 206 |
| 5115-H4-M34-14 | 89 | 129 | 134 | 186 | 195 | 202 | 206 |
| 5115-H4-M34-15 | 95 | 103 | 134 | 157 | 195 | 202 | 206 |
| 5115-H4-M34-16 | 85 | 124 | 134 | 157 | 195 | 202 | 206 |

For example, the antigen-binding protein described herein may include an antigen-binding protein (e.g., a single domain antibody) having the same HCDR3 (e.g., having the same HCDR1-3) as the aforementioned antigen-binding protein. In some cases, the antigen-binding protein may include an antigen-binding protein (e.g., a single domain antibody) having the same VH as the aforementioned antigen-binding protein.

For example, the antigen-binding protein may include a variable domain of the heavy chain (VH), and the VH may include the amino acid sequence set forth in SEQ ID NO: 225 (EVQLVES [GS] GGLVQ [AP] G[DG] SLRL SCA[AV] S [GHLMRST] [AGIKPRTV] [AEFGPRST Y] [FLR] [KNPRS] [GRST] [FLY] V [LM] [AGMS] WFRQAPGK[EG] [LR]E[FL] V [AS]AISG[FGH KQRST]G[IKLMQRST][QRV][AFHLMQSTVY][LNRTY]YADSVKGRF[AT][IV]SRDN[AS] [KN]NT[LV]YLQM[NS]SL[KR][AP][DE]DT[AG][LV]YYCAANPR[RY]AT[PT][DG][FLMY ][HIKLMQRST][AQRS]Y[AKL]YWGQGT[LQ]VTVSS) or 226 (EVQLVES [GS] GGLVQ [AP] G[DG] SLRL SCA[AV] S [GHLMRST] [AGIKPRTV] [AEFGPRST Y] [FLR] [KNPRS] [GRST] [FLY] V [LM] [AGMS] WFRQAPGK[EG] [LR]E[FL] V [AS]AISG[FGH KQRST]G[IKLMQRST][QRV][AFHLMQSTVY][LNRTY]YADSVKGRF[AT][IV]SRDN[AS] [KN]NT[LV]YLQM[NS]SL[KR][AP][DE]DT[AG][LV]YYCAANPR[RY]AT[PT][DG][FLMY ][HIKLMQRST][AQRS]Y[AKL]YWGQGT[LQ]VTVSS). The letters in square brackets indicate the type of an amino acid that may be selected at that site.

For example, the antigen-binding protein may include a variable domain of the heavy chain (VH), and the VH may include the amino acid sequence set forth in any one of SEQ ID NOs: 1-80.

For example, the antigen-binding protein may include HCDR1, HCDR2 and HCDR3 of a variable domain of the heavy chain (VH), and the VH may include the amino acid sequence set forth in any one of SEQ ID NOs: 1-80.

For example, the antigen-binding protein may include an antibody or an antigen-binding fragment thereof.

For example, the antigen-binding protein may include a single domain antibody or an antigen-binding fragment thereof.

For example, the antigen-binding protein may be selected from the group consisting of Fab, Fab', F(ab)2, Fv fragment, F(ab')2, scFv, di-scFv, VHH and/or dAb.

For example, the antibody may include an alpaca antibody, a chimeric antibody, a humanized antibody, and/or a fully human antibody.

For example, the antigen-binding protein may include the amino acid sequence set forth in any one of SEQ ID NOs: 1-80.

The present application provides a polypeptide, which may include the antigen-binding protein described herein.

For example, the polypeptide may further include a structure that improves the stability of the antigen-binding protein and/or is capable of binding to an Fc receptor.

For example, the polypeptide may further include an immunoglobulin Fc region.

For example, the antigen-binding protein may be directly or indirectly linked to the Fc region.

For example, the C-terminus of the VH of the antigen-binding protein may be directly or indirectly linked to the N-terminus of the Fc region.

For example, the Fc region may include Fc derived from IgG1 and/or Fc derived from IgG4.

For example, the Fc region may include the amino acid sequence set forth in SEQ ID NO: 227 (EPKS[ACS]DKTHTCPPCPAPE[AL][AL]GGPSVFLFPPKPKDTLMISRTPEVTCVVVDVSH EDPEVKFNWYVDGVEVHNAKTKPREEQYNSTYRVVSVLTVLHQDWLNGKEYKCKVSN KALPAPIEKTISKAKGQPREPQVYTLPPSR[DE]E[LM]TKNQVSLTCLVKGFYPSDIAVEW ESNGQPENNYKTTPPVLDSDGSFFLYSKLTVDKSRWQQGNVFSCSVMHEALHNHYTQK SLSLSPGK). The letters in square brackets indicate the type of an amino acid that may be selected at that site.

For example, the Fc region may include the amino acid sequence set forth in any one of SEQ ID NOs: 209-212.

The present application provides an immunoconjugate, which may include the isolated antigen-binding protein described herein and/or the polypeptide described herein.

The present application provides a nucleic acid, which may encode the isolated antigen-binding protein described herein and/or the polypeptide described herein.

The nucleic acid described herein may be isolated. For example, the nucleic acid may be produced or synthesized by: (i) amplification in vitro, e.g., by polymerase chain reaction (PCR), (ii) clonal recombination, (iii) purification, e.g., by enzymatic digestion and gel electrophoresis fractionation, or (iv) synthesis, e.g., by chemical synthesis. For example, the isolated nucleic acid may be a nucleic acid molecule prepared by recombinant DNA technology. In the present application, the nucleic acid encoding the isolated antigen-binding protein may be prepared by various methods known in the art, including but not limited to, reverse transcription PCR and PCR, to obtain the nucleic acid molecule of the isolated antigen-binding protein described herein.

The present application provides an expression vector, which may include the nucleic acid described herein. Each vector may include one or more of the nucleic acid molecules. In addition, the vector may further include another gene, for example, a marker gene that allows selection of the vector in an appropriate host cell and under an appropriate condition. In addition, the vector may further include an expression control element that allows correct expression of a coding region in an appropriate host. Such a control element is well known to those skilled in the art, and may include, for example, a promoter, a ribosome binding site, an enhancer, and other control elements that regulate gene transcription or mRNA translation. In some embodiments, the expression control sequence is a regulatable element. The specific structure of the expression control sequence may vary depending on species or the function of cell types, but generally includes a 5' non-transcribed sequence and 5' and 3' non-translated sequences involved in the initiation of transcription and translation, respectively, for example, a TATA box, a caped sequence, and a CAAT sequence. For example, the 5' non-transcribed expression control sequence may include a promoter region, and the promoter region may include a promoter sequence for transcriptional control of a functionally linked nucleic acid. The expression control sequence may further include an enhancer sequence or an upstream activator sequence. In the present application, a suitable promoter may include, for example, a promoter for SP6, T3 and T7 polymerases, a human U6 RNA promoter, a CMV promoter and an artificial hybrid promoter thereof (e.g., CMV), wherein a certain moiety of the promoter may be fused with a certain moiety of the gene promoter of another cellular protein (e.g., human GAPDH, and glyceraldehyde-3-phosphate dehydrogenase). The promoter may or may not include an additional intron. One or more nucleic acid molecules described herein may be operably linked to the expression control element.

The vectors may include, for example, a plasmid, a cosmid, a virus, a phage, or other vectors commonly used in, for example, genetic engineering. For example, the vector may be an expression vector. For example, the vector may be a viral vector. The viral vector may be administered directly to a patient (in vivo) or may be administered indirectly, for example, by treating a cell with the virus in vitro and then administering the treated cell to a patient (ex vivo). Viral vector technology is well known in the art and described in, for example, Handbooks of Virology and Molecular Biology. A conventional viral-based system may include a retroviral vector, a lentiviral vector, an adenoviral vector, an adeno-associated viral vector, and a herpes simplex viral vector for gene transfer. In some cases, retroviral, lentiviral, and adeno-associated viral methods may be used to integrate gene transfer into a host genome, allowing for long-term expression of the inserted gene. A lentiviral vector is a retroviral vector capable of transducing or infecting a non-dividing cell and typically producing a higher viral titer. A lentiviral vector may include a long terminal repeat sequence 5'LTR and a truncated 3 'LTR, RRE, a rev response element (cPPT), a central termination sequence (CTS), and/or a post-translational regulatory element (WPRE). The vector described herein may be introduced into a cell.

The present application provides a host cell, which may include the isolated antigen-binding protein described herein, the polypeptide described herein, the immunoconjugate described herein, the nucleic acid described herein, and/or the vector described herein. The cell may include, the isolated antigen-binding protein, the polypeptide, the immunoconjugate, one or more nucleic acid molecules, and/or one or more vectors, described herein. For example, each type of or each cell may include one or more nucleic acid molecules or vectors described herein. For example, each type of or each cell may include multiple (e.g., 2 or more) or multiple types (e.g., 2 or more) of the nucleic acid molecules or vectors described herein. For example, the vector described herein may be introduced into the host cell, such as a prokaryotic cell (e.g., a bacterial cell), a CHO cell, a NS/0 cell, a HEK293T cell, a 293F cell or a HEK293A cell, or other eukaryotic cells, such as cells from plants, fungi or yeast cells. The vector described herein may be introduced into the host cell by methods known in the art, for example, electroporation, lipofectine transfection, and lipofectamin transfection. For example, the cell may include a yeast cell. For example, the cell may include an *E.coli* cell. For example, the cell may include a mammalian cell. For example, the cell may include an immune cell.

A method for preparing the isolated antigen-binding protein described herein and/or the polypeptide described herein is provided, and includes culturing the cell described herein under a condition of allowing expression of the isolated antigen-binding protein and/or the polypeptide.

A composition is provided, and may include the isolated antigen-binding protein described herein, the polypeptide described herein, the immunoconjugate described herein, the nucleic acid described herein, the vector described herein, and/or the cell described herein, and optionally a pharmaceutically acceptable adjuvant. In the present application, the pharmaceutically acceptable adjuvant may include a buffer, an antioxidant, a preservative, a low molecular weight polypeptide, a protein, a hydrophilic polymer, an amino acid, a sugar, a chelating agent, a counterion, a metal complex, and/or a nonionic surfactant. Unless incompatible with the cell described herein, any conventional medium or agent is contemplated for use in the pharmaceutical composition described herein. In the present application, the pharmaceutically acceptable excipient may include an additive other than the main drug in a pharmaceutical preparation, and may also be referred to as an auxiliary material.

The present application provides a kit, which may include the isolated antigen-binding protein described herein, the polypeptide described herein, the immunoconjugate described herein, the nucleic acid described herein, the vector described herein, the cell described herein, and/or the composition described herein.

The present application provides a method for inhibiting proliferation of cells overexpressing IGF-1R or a functionally active fragment thereof, and the method may include administering the isolated antigen-binding protein described herein, the polypeptide described herein, the immunoconjugate described herein, the nucleic acid described herein, the vector described herein, the cell described herein, the composition described herein, and/or the kit described herein. The present application provides a inhibiting an MAPK pathway, which may include administering the isolated antigen-binding protein described herein, the polypeptide described herein, the immunoconjugate described herein, the nucleic acid described herein, the vector described herein, the cell described herein, the composition described herein, and/or the kit described herein. For example, the inhibiting an MAPK pathway may include inhibiting phosphorylation of an MAPK pathway protein. For example, the MAPK pathway protein may include MEK, Erk1/2 and Akt, or functionally active fragments thereof. In the present application, the method may include an in vitro method, an ex vivo method, or a method for a non-diagnostic or non-therapeutic purpose.

The present application provides a method for inhibiting interaction between IGF-1R or a functionally active fragment thereof and ligands thereof, and the method may include administering the isolated antigen-binding protein described herein, the polypeptide described herein, the immunoconjugate described herein, the nucleic acid described herein, the vector described herein, the cell described herein, the composition described herein, and/or the kit described herein. For example, the ligands may include IGF-1 or functionally active fragments thereof. In the present application, the method may include an in vitro method, an ex vivo method, or a method for a non-diagnostic or non-therapeutic purpose.

The present application provides a method for detecting the presence and/or content of IGF-1R or a functionally active fragment thereof, and the method may include administering the isolated antigen-binding protein described herein, the polypeptide described herein, the immunoconjugate described herein, the nucleic acid described herein, the vector described herein, the cell described herein, the composition described herein, and/or the kit described herein. In the present application, the method may include an in vitro method, an ex vivo method, or a method for a non-diagnostic or non-therapeutic purpose. For example, the presence and/or expression level of IGF-1R in a sample obtained from a subject may be determined by contacting the sample with the antigen-binding protein described herein; and detecting the presence and/or content of the antigen-binding protein bound to the sample.

The present application provides use of the isolated antigen-binding protein described herein, the polypeptide described herein, the immunoconjugate described herein, the nucleic acid described herein, the vector described herein, the cell described herein, the composition described herein, and/or the kit described herein in preparation of drugs for preventing, alleviating, and/or treating diseases or disorders. For example, the diseases or disorders may include tumors. For example, the diseases or disorders may include tumors associated with expression of IGF-1R or a functionally active fragment thereof. For example, the diseases or disorders may include breast cancer.

The present application provides the isolated antigen-binding protein described herein, the polypeptide described herein, the immunoconjugate described herein, the nucleic acid described herein, the vector described herein, the cell described herein, the composition described herein, and/or the kit described herein for preventing, alleviating, and/or treating diseases or disorders. For example, the diseases or disorders may include tumors. For example, the diseases or disorders may include tumors associated with expression of IGF-1R or a functionally active fragment thereof. For example, the diseases or disorders may include breast cancer.

The present application provides a method for preventing, alleviating, and/or treating diseases or disorders, and the method includes administering an effective dose of the isolated antigen-binding protein described herein, the polypeptide described herein, the immunoconjugate described herein, the nucleic acid described herein, the vector described herein, the cell described herein, the composition described herein, and/or the kit described herein to a subject in need. For example, the diseases or disorders may include tumors. For example, the diseases or disorders may include tumors associated with expression of IGF-1R or a functionally active fragment thereof. For example, the diseases or disorders may include breast cancer.

Not limited by any theory, the examples described below are only intended to illustrate the protein, the preparation method and the use described herein, and are not intended to limit the scope of invention of the present application.

### Examples

### Example 1 Immunization of alpaca

A healthy adult alpaca (Alpaca) was immunized with recombinant human IGF-1R proteins (his tag). For initial immunization, 0.5 mg of the recombinant human IGF-1R proteins was emulsified with an equal volume of Freund's complete adjuvant and injected subcutaneously at multiple sites. For booster immunization, 0.25 mg of the recombinant human IGF-1R proteins was emulsified with an equal volume of Freund's incomplete adjuvant and injected subcutaneously at multiple sites. A total of 3 booster immunizations were conducted. Blood was collected before the initial immunization and one week after each booster immunization to monitor the antiserum titer.

### Example 2 Construction of alpaca immune library

Following immunization, peripheral blood was collected from the alpaca, and lymphocytes were separated using a lymphocyte separation medium. Total RNA was extracted using a TRIzol^{™} Reagent. cDNA was obtained by reverse transcription using a SuperScript^{®} III First-Strand Synthesis System for RT-PCR, and VHH genes were amplified by nested PCR. The VHH gene fragments were recovered by a gel purification kit, digested with restriction endonuclease sfiI, and then cloned into phagemid vectors pComb 3XSS. The constructed cloned product was transformed into *E.coli* TG1 electroporation competent cells to construct a VHH gene library. The library capacity was 1.3×10⁹ cfu determined by a plate gradient dilution method. Colony PCR results showed that the library insertion rate was 100%. The gene library was inoculated and cultured to the logarithmic phase, and then rescued using M13K07 phages. Following the rescue culture, the phages were collected by centrifugation and purified using PEG-NaCl to obtain a phage display library for subsequent screening.

### Example 3 Screening and identification of nanobodies

The nanobody phage display library constructed in Example 2 was incubated upside down in a 1% casein-blocked immunotube (Nunc-lmmuno^{™}) at room temperature for 90 min, and then allowed to stand for 30 min to remove non-specifically bound phages. The library was then transferred to an immunotube coated with IGF-1R (hIGF-1R-his) antigens, incubated upside down at room temperature for 90 min, and then allowed to stand for 30 min. The immunotube was washed 10 times with PBST to remove non-bound or low-binding phages. The specific-bound phages were eluted by adding 1 mL of freshly prepared 100 mM triethylamine, and neutralized by addition of 500 µL of 1M Tris-HCl, pH 6.4, and the eluent was collected and placed on ice after slight shaking. *E.coli* TG1 in the logarithmic growth phase was infected with the eluate, and the infected *E.coli* TG1 was serially diluted and spread on an LB solid culture medium (by a plate dilution method). After 12-14 h, plate counting was conducted, the amount of eluted phages was calculated, and single clones were randomly picked and amplified to obtain phages, which were identified by an enzyme-linked immunosorbent assay (ELISA). The method included: a 96-well Greiner ELISA plate was coated using 50 nM recombinant human IGF-1R (hIGF 1R-his) proteins overnight at 4°C, and then blocked with 1% casein. The phages to be identified were blocked with 0.1% casein for 1 h and then added to the above ELISA plate for incubation for 2 h, followed with PBST washing between different blocking/incubations. Following washing, anti-M13-HRP (Sinobio, 11973-MM05) was added and allowed to bind at 37°C for 1 h. Following washing, a TMB substrate solution (Thermo, 34029) was added for color development. Following termination of the reaction, the OD₄₅₀ was read using a microplate reader. Positive control wells (an enriched library in each round), a negative control (helper phages), and blank wells (PBS) were set. If the OD value of the test wells was more than 3 times that of the negative control, the test wells were judged as positive. The clones corresponding to the positive wells were cultured, extracted to obtain plasmids, and sequenced. The DNA sequences were translated into amino acid sequences and compared. Clones with the same CDR1, CDR2 and CDR3 sequences were considered to be the same antibody strains, and clones with different CDR sequences were considered to be different antibody strains. A total of 43 unique sequences were obtained.

TG1 with a unique sequence identified by the above ELISA binding experiment was induced overnight with 1 µM IPTG, and following repeated freezing and thawing, the supernatant was collected by centrifugation. Following blocking with 0.1% BSA for 1 h, the supernatant sample and 1.25 µg/mL IGF1-Fc were added to the ELISA plate coated with 5 µg/mL antigen proteins and blocked with 1% casein, and were incubated at room temperature for 1 h. The plate was washed with PBST between different incubations. Following addition of anti-human IgG-HRP (Abcam ab6759, diluted 1:5,000 in 1% casein) and incubation for 1 h, the cells were washed three times with PBST, and a TMB substrate solution (Thermo, 34029) was added for color development. Following termination of the reaction, the OD₄₅₀ was read using a microplate reader. The blocking activity of the clones was ranked according to the results, and some of the sequences with the highest blocking function were selected for subsequent purification and expression.

### Example 4 expression and purification of VHHs of nanobodies

The positive clones with unique sequences and a strong inhibitory effect screened out in Example 3 were transformed into Top10F', and the expression of VHHs of nanobodies was induced using IPTG. The cell pellet was collected by centrifugation and the supernatant was collected following ultrasonic disruption. The supernatant was purified using TALON^{®} Metal Affinity Resins (Takara, 635502) and the buffer was replaced with a phosphate buffer. The purified VHHs were quantified by measuring the OD₂₈₀ and stored at 4°C until use.

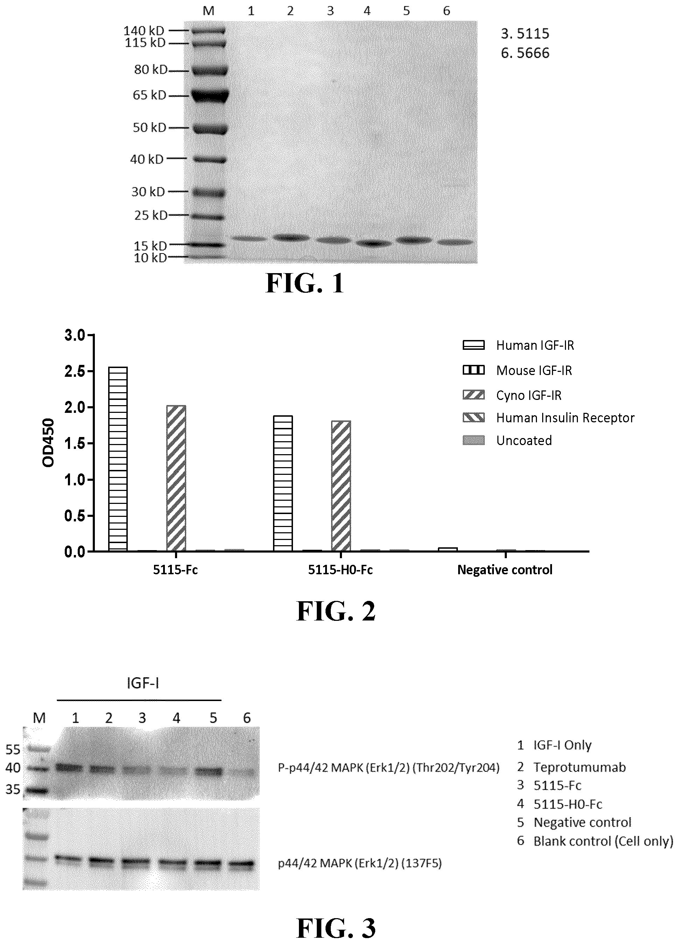

The SDS-PAGE of the purified nanobodies is shown in FIG. 1.

### Example 5 ELISA experiment on nanobodies in competitive binding to antigen proteins and ligands thereof

Recombinant human IGF-1R-his antigens (5 µg/mL) were added to a 96-well plate for coating overnight at 4°C. The plate was washed with PBS, and then was blocked with 1% casein at room temperature for 1 h. The VHH samples were diluted 2-fold serially with 0.1% BSA/PBS, with a starting concentration of 20 µg/mL. VHHs of different concentrations and 1.25 µg/mL IGF-1-Fc were added to the antigen-coated 96-well plate and incubated at room temperature for 1 h. The plate was washed three times with 0.1% PBST, horseradish peroxidase-labeled anti-human Fc tag antibodies (anti-human IgG-HRP, Abcam ab6759, 1:5,000, containing 1% casein) were added and incubated at room temperature for 30 min, and the plate was washed three times with 0.1% PBST. A TMB substrate solution (Thermo, 34028) was added for color development, the OD₄₅₀ was read using a microplate reader, and a curve was fit and the EC₅₀ value was calculated using Graphpad Prism software. The results showed that multiple nanobodies could effectively compete to bind to human IGF-1R, and the IC₅₀ values of two nanobodies are shown in Table 1.

**Table 1 IC₅₀ values of nanoantibodies in competitive binding to IGF-1-Fc and IGF-1R**

| ID | IC₅₀ (µg/mL) |
|---|---|
| 5115 | 0.24 |
| 5666 | 0.70 |

### Example 6 Expression and purification of nanobody-Fc fusion proteins

pTT5 vectors including nucleic acid sequences encoding the nanobodies and human Fc segments (the C-termini of VHHs were linked to the N-termini of human Fcs) were synthesized, and a liposome-DNA complex was prepared by the following steps: 15 µg of plasmid DNA was diluted with Opti-MEM^{®} I (GIBCO 31985) to a total volume of 0.5 mL; 30 µL of a 293fectin^{™} reagent was diluted with Opti-MEM^{®} I to a total volume of 0.5 mL; and the dilutions were gently mixed and incubated at room temperature for 5 min. The diluted DNA was added to a diluted 293fectin^{™} (Invitrogen 12347) reagent, mixed gently, and incubated at room temperature for 30 min. The resulting mixture was added to a shaking flask containing 1×10⁶ cells/mL (FreeStyle^{™} 293F Cells, Invitrogen R790-07) and incubated on an orbital shaker rotating at 125 rpm in a cell culture incubator at 37°C and 8% CO₂. On day 4 following transfection, the supernatant was harvested and purified using Protein A agarose gel of Pierce^{™}, and the buffer was replaced with a phosphate buffer.

### Example 7 ELISA experiment on nanobody-Fc fusion proteins in competitive binding to antigen proteins and ligands thereof

Recombinant human IGF1R-his antigen (5 µg/mL) was added to a 96-well plate for coating overnight at 4°C. The plate was washed 4 times with 1×TBST, and then was blocked with 2% BSA at room temperature for 1.5 h. The plate was washed four times with 1×TBST, and 50 µL of nanobody-Fc fusion proteins of different concentrations (with a starting concentration of 10 µg/mL, 2-fold serially diluted) and 50 µL of biotinylated IGF-1 (2.5 µg/mL) were added to the antigen-coated 96-well plate and incubated at 37°C for 1 h. The plate was washed 4 times with 1×TBST, horseradish peroxidase-labeled Streptavidin (Thermo, 21126) was added and incubated at 37°C for 1 h, and the plate was washed 4 times with 1×TBST. TMB substrate solution was added at 200 µL per well for color development in the dark for 20 min, 50 µL of 1 M H₂SO₄was added to terminate the reaction, and the OD₄₅₀ was read using a microplate reader. A curve was fit and the IC₅₀ value was calculated using Graphpad Prism software. As shown in Table 2, IGF 1R-5115 Fc could effectively compete to bind to IGF-1R, and had an inhibitory activity higher than that of a positive control antibody Teprotumumab.

**Table 2 IC₅₀ values of nanoantibody-Fc fusion proteins competing with IGF-1 to bind to IGF-1R**

| ID | IC₅₀ (µg/mL) |
|---|---|
| 5115 Fc | 0.435 |
| Teprotumumab | 0.986 |

### Example 8 Humanization of nanobodies and detection of binding activity of humanized nanobody-Fc fusion proteins to antigen proteins

The aforementioned nanobodies 5115 were selected and humanized. By performing database sequence analysis on sequences, the IGHV3-23 germline gene was selected as the template to conduct CDR transplantation (while retaining some of alpaca amino acids in sensitive sites) to obtain 5115-H0. On the basis of 5115-H0, combinatorial back mutation design was further conducted based on experience, and a total of 6 humanized sequences: 5115-H1, 5115-H2, 5115-H3, 5115-H4, 5115-H5, and 5115-H6 were obtained. The humanized VHH gene sequences and the Fc sequences were synthesized into full-length sequences. pcDNA3.4 plasmids were digested with NotI/XbaI (NEB, Cat. R0189L, R0145L) and recombined with the above full-length sequences. The recombinant plasmids were transformed into TOP10 competent cells and bacterial solutions were spread and cultured at 37°C for 16-20 h. Single colonies on the plate were picked to conduct colony PCR and electrophoresis to identify positive clones, the positive clones were picked and sequenced, and the clones with correct sequencing were expanded for culture and extracted to obtain plasmids. Using an ExpiCHO-S expression system, the plasmids were expressed in ExpiCHO-S cells for 7 d as required. Finally, the VHH-Fc fusion proteins were obtained by one-step purification by Protein A affinity chromatography.

Recombinant human IGF-1R-his antigens (2 µg/mL) were added to a 96-well plate for coating overnight at 4°C. The plate was washed three times with PBST, and then was blocked with PBST containing 5% skimmed milk powder at room temperature for 2 h. The blocking solution was discarded, the plate was washed three times with PBST, and then 30 µL of nanobody-Fc fusion proteins of different concentrations were added and incubated at room temperature for 1 h. The plate was washed 3 times with PBST, horseradish peroxidase-labeled anti-human Fc antibodies (abcam, ab97225) were added at 30 µL/well and incubated at room temperature for 1 h, and the plate was washed 3 times with PBST. TMB substrate solution was added at 30 µL/well for color development in the dark for 5-30 min, 30 µL of 2 M H₂SO₄ was added to terminate the reaction, and the OD₄₅₀ was read using a microplate reader. A curve was fit and the EC₅₀ value was calculated using Graphpad Prism software. The results showed that the aforementioned nanobody-Fc fusion proteins may effectively bind to the antigen proteins, and the binding activity of some of humanized nanobodies is comparable to or higher than that of the positive control. The EC₅₀ values of the samples were shown in Table 3.

**Table 3 EC₅₀ values of nanobody-Fc fusion proteins binding to antigens**

| Name | EC₅₀ (µg/mL) |
|---|---|
| 5115-H0-Fc | 0.0327 |
| 5115-H1-Fc | 0.0369 |
| 5115-H2-Fc | 0.0652 |
| 5115-H3-Fc | 0.0925 |
| 5115-H4-Fc | 0.0828 |
| 5115-H6-Fc | 0.0929 |
| Teprotumumab | 0.1442 |

### Example 9 Species-cross validation and selective binding validation of nanobody-Fc fusion proteins to antigen proteins

Recombinant human, monkey and mouse IGF-1R-his antigens and human insulin receptor proteins (5 µg/mL) were added to a 96-well plate for coating overnight at 4°C. The plate was washed with PBS, and then was blocked with 1% casein at room temperature for 1 h. The nanobody-Fc fusion proteins were diluted to 2 µg/mL with 0.1% BSA/PBS, added to the antigen-coated 96-well plate, and incubated at room temperature for 1 hour. The plate was washed three times with 0.1% PBST, horseradish peroxidase-labeled anti-human Fc tag antibodies (anti-human IgG-HRP, Abcam ab6759, 1:5,000, containing 1% casein) were added and incubated at room temperature for 30 min, and the plate was washed three times with 0.1% PBST. A TMB substrate solution (Thermo, 34028) was added for color development, and the OD₄₅₀ was read using a microplate reader.

As shown in FIG. 2, the 5115-Fc fusion proteins and the humanized nanobody-Fc fusion proteins (5115-H0 Fc) had a binding activity to both recombinant human IGF-1R and cynomolgus monkey IGF-1R, but had no binding activity to mouse IGF-1R. In addition, the two tested nanobody-Fc fusion proteins had no binding activity to the human insulin receptor proteins, indicating good selectivity for the antigen IGF-1R.

### Example 10 Construction of mutants of humanized nanobody-Fc fusion proteins and detection of binding activity thereof to antigen proteins

First-round mutant construction: 5115-H4 was selected as a parent, and the VHH genes thereof were linked to dAb phage display vectors to obtain parent plasmids. A series of primers were designed to conduct single- or double-point mutations on the parents, and 4 affinity matured phage display libraries were constructed. Immunotube screening (i.e., solid phase screening) was conducted, the immunotube was coated with the antigen proteins recombinant human IGF-1R-His, the aforementioned affinity matured phage display libraries were added for incubation, washing and elution, and three rounds of panning were conducted to enrich high-affinity specific monoclonal antibodies. After the panning process and VHH ELISA, 15 candidate molecules were selected to conduct expression of nanobody-Fc fusion proteins and ELISA based on the affinity and sequence similarity at the VHH level.

Second-round mutant construction: 5115-H4-M34 and 5115-H1 were selected respectively as parents, and the VHH genes thereof were linked to dAb phage display vectors to obtain parent plasmids. A series of primers were designed to conduct single- or double-point mutations on the parents, and a total of 8 affinity matured phage display libraries were constructed. Immunotube screening (i.e., solid phase screening) was conducted, the immunotube was coated with the antigen proteins hIGF 1R-His, the aforementioned affinity matured phage display libraries were added for incubation, washing and elution, and three rounds of panning were conducted to enrich high-affinity specific monoclonal antibodies. After the panning process and VHH ELISA, based on the affinity and sequence similarity at the VHH level, 16 candidate molecules were selected from the mutants with the 5115-H4-M34 as the parent to conduct expression of nanobody-Fc fusion proteins, and 40 candidate molecules were selected from the mutants with the 5115-H1 as the parent to conduct expression of nanobody-Fc fusion proteins and ELISA.

The binding activity of the nanobody-Fc fusion proteins obtained after two rounds of mutant construction with the antigen proteins (recombinant human IGF-1R-His) was detected by an ELISA method: 2 µg/mL recombinant human IGF 1R-His antigen proteins were added to a 96-well ELISA plate, at 30 µL/well, for coating overnight at 4°C. The plate was washed three times with PBST. PBS containing 5% skim milk powder was added for blocking at room temperature for 2 h. The plate was washed three times with PBST. The nanobody-Fc fusion proteins were serially diluted 3-fold, and the nanobody-Fc fusion proteins of different concentrations were added to each well at 30 µL/well and incubated at room temperature for 1 h. The plate was washed three times with PBST, and horseradish peroxidase-labeled anti-human Fc antibodies were added at 30 µL/well and incubated at room temperature for 50 min. The plate was washed three times with PBST. TMB substrate solution was added at 30 µL/well for color development in the dark for 1-5 min, 30 µL of 2 M hydrochloric acid was added to terminate the reaction, and the OD₄₅₀ was read using a microplate reader. Curve fitting was conducted and the EC₅₀ value was calculated using Graphpad Prism software. As shown in Tables 4 and 5, the nanobody-Fc fusion proteins obtained by the mutant construction all had high affinity to the antigens, and the binding activity of some nanobody fusion proteins to the antigens is comparable to or higher than that of the positive control Teprotumumab.

**Table 4 EC₅₀ value of nanobody-Fc fusion proteins, obtained by the first round of mutant construction, binding to antigens**

| Name | EC₅₀ (nM) | Name | EC₅₀ (nM) |
|---|---|---|---|
| 5115-H4-M3-Fc | 0.330 | 5115-H4-M41-Fc | 0.175 |
| 5115-H4-M10-Fc | 0.094 | 5115-H4-M44-Fc | 0.170 |
| 5115-H4-M12-Fc | 0.202 | 5115-H4-M48-Fc | 0.475 |
| 5115-H4-M24-Fc | 0.332 | 5115-H4-M52-Fc | 0.183 |
| 5115-H4-M25-Fc | 0.215 | 5115-H4-M59-Fc | 0.950 |
| 5115-H4-M28-Fc | 0.299 | 5115-H4-M61-Fc | 0.107 |
| 5115-H4-M34-Fc | 0.052 | 5115-H4-M69-Fc | 0.118 |
| 5115-H4-M39-Fc | 0.210 | Teprotumumab | 0.057 |

**Table 5 EC₅₀ value of nanobody-Fc fusion proteins, obtained by the second round of mutant construction, binding to antigens**

| Name | EC₅₀ (ng/mL) | Name | EC₅₀ (ng/mL) | Name | EC₅₀ (ng/mL) |
|---|---|---|---|---|---|
| 5115-H4-M34-1-Fc | 3.918 | 5115-H1-M4-Fc | 1.939 | 5115-H1-M23-Fc | 1.740 |
| 5115-H4-M34-2-Fc | 3.328 | 5115-H1-MS-Fc | 5.880 | 5115-H1-M24-Fc | 1.831 |
| 5115-H4-M34-3-Fc | 3.639 | 5115-H1-M6-Fc | 2.622 | 5115-H1-M25-Fc | 1.329 |
| 5115-H4-M34-4-Fc | 6.241 | 5115-H1-M7-Fc | 3.038 | 5115-H1-M26-Fc | 1.523 |
| 5115-H4-M34-5-Fc | 2.147 | 5115-H1-M8-Fc | 1.997 | 5115-H1-M27-Fc | 1.786 |
| 5115-H4-M34-6-Fc | 3.499 | 5115-H1-M9-Fc | 2.301 | 5115-H1-M28-Fc | 1.182 |
| 5115-H4-M34-7-Fc | 5.363 | 5115-H1-M10-Fc | 1.699 | 5115-H1-M29-Fc | 1.618 |
| 5115-H4-M34-8-Fc | 3.794 | 5115-H1-M11-Fc | 2.508 | 5115-H1-M30-Fc | 2.448 |
| 5115-H4-M34-9-Fc | 4.053 | 5115-H1-M12-Fc | 1.414 | 5115-H1-M31-Fc | 1.841 |
| 5115-H4-M34-10-Fc | 3.708 | 5115-H1-M13-Fc | 2.903 | 5115-H1-M32-Fc | 1.577 |
| 5115-H4-M34-11-Fc | 2.334 | 5115-H1-M14-Fc | 4.306 | 5115-H1-M33-Fc | 1.152 |
| 5115-H4-M34-12-Fc | 3.329 | 5115-H1-M15-Fc | 2.668 | 5115-H1-M34-Fc | 2.054 |
| 5115-H4-M34-13-Fc | 2.435 | 5115-H1-M16-Fc | 3.611 | 5115-H1-M35-Fc | 1.466 |
| 5115-H4-M34-14-Fc | 3.275 | 5115-H1-M17-Fc | 1.844 | 5115-H1-M36-Fc | 1.427 |
| 5115-H4-M34-15-Fc | 2.253 | 5115-H1-M18-Fc | 2.893 | 5115-H1-M38-Fc | 1.661 |
| 5115-H4-M34-16-Fc | 2.405 | 5115-H1-M19-Fc | 1.925 | 5115-H1-M39-Fc | 1.060 |
| 5115-H1-M1-Fc | 1.219 | 5115-H1-M20-Fc | 2.206 | 5115-H1-M40-Fc | 1.227 |
| 5115-H1-M2-Fc | 1.499 | 5115-H1-M21-Fc | 3.349 | Teprotumumab | 2.202 |
| 5115-H1-M3-Fc | 1.218 | 5115-H1-M22-Fc | 1.791 | | |

### Example 11 Detection of affinity of nanoantibody-Fc fusion proteins to recombinant human IGF-1R

The affinity of the nanobody fusion proteins to recombinant human IGF-1R was detected using a BIAcore instrument. The antigens were diluted to 12.5 µg/mL with an NaAc buffer of pH 4.75. The flow rate was set to 10 µL/min. The chip activation time of a mixture of EDC and NHS was the default value of 420 s. The recombinant human IGF-1R-his was coupled to 800 RU on a CM5 chip under the preset coupling mode, and unbound activated groups were blocked with ethanolamine. Two-fold serially diluted nanobody-Fc fusion proteins (five concentrations of each fusion protein were detected) were injected sequentially at a flow rate of 50 µL/min at 25°C, with an association time of 120 s and a dissociation time of 600 s. A curve was fit with a 1:1 Langmuir binding model using the BIAcore T200 analysis software to determine kinetic constants such as the association rate constant, the dissociation rate constant, and the association dissociation constant.

The results showed that the tested nanobody-Fc fusion proteins all had high affinity to recombinant human IGF-1R, and the affinity of some nanobody-Fc fusion proteins to the antigens was comparable to or higher than that of the positive control. The kinetic constants of the nanobody-Fc fusion proteins binding to the recombinant human IGF-1R-his were shown in Table 6.

**Table 6 Kinetic constants of nanoantibody-Fc fusion proteins binding to recombinant human IGF-1R his**

| Test 1: | | | |
|---|---|---|---|
| Name | ka (1/Ms) | kd (1/s) | K_{D} |
| 5115-H1-Fc | 6.68e+04 | 2.11e-03 | 3.17e-8 |
| 5115-H1-M3-Fc | 1.12e+06 | 2.07e-03 | 1.85e-9 |
| 5115-H1-M10-Fc | 1.07e+06 | 1.23e-03 | 1.15e-9 |
| 5115-H1-M17-Fc | 4.62e+06 | 4.65e-04 | 1.01e-10 |
| 5115-H4-M34-Fc | 3.18e+06 | 2.74e-04 | 8.62e-11 |
| 5115-H4-M34-13-Fc | 2.24e+06 | 3.37e-04 | 1.51e-10 |
| Teprotumumab | 1.32e+06 | 4.10e-04 | 3.11e-10 |

| Test 2: | | | |
|---|---|---|---|
| Name | ka (1/Ms) | kd (1/s) | K_{D} |
| 5115-H4-M44-Fc | 6.16e+06 | 1.26e-03 | 2.05e-10 |
| 5115-H4-M61-Fc | 5.53e+06 | 1.92e-03 | 3.47e-10 |
| 5115-H4-M69-Fc | 7.80e+06 | 1.26e-03 | 1.61e-10 |
| Teprotumumab | 1.99e+06 | 4.56e-04 | 2.29e-10 |

### Example 12 ELISA experiment on nanobody-Fc fusion proteins in competitive binding to antigen proteins and ligands thereof

Nanobody-Fc fusion proteins with a higher antigen binding activity were selected from humanized and mutant libraries to verify the activity thereof in competitive binding to the antigen proteins and ligands thereof.

Recombinant human IGF-1R-his antigens (5 µg/mL) were added to a 96-well plate for coating overnight at 4°C. The plate was washed 4 times with 1×TBST, and then was blocked with 2% BSA at 37°C for 1.5 h. The plate was washed four times with 1×TBST. A volume of 50 µL of biotinylated IGF-1-Fc (2.5 µg/mL) or IGF-2 (N-Avi & N-Fc 4 µg/mL), and 50 µL of nanobody-Fc fusion proteins of different concentrations (with a starting concentration of 10 µg/mL, 2-fold serially diluted) were added to the 96-well plate and incubated at 37°C for 1 h. The plate was washed 4 times with 1×TBST, horseradish peroxidase-labeled Streptavidin (Thermo, 21126) was added and incubated at 37°C for 1 h, and the plate was washed 4 times with 1×TBST. TMB substrate solution was added at 200 µL/well for color development in the dark for 20 min, 50 µL of 1 M H₂SO₄ was added to terminate the reaction, and the OD₄₅₀ was read using a microplate reader. A curve was fit and the IC₅₀ value was calculated using Graphpad Prism software. As shown in Table 7, the nanoantibody-Fc fusion proteins could effectively compete to bind to IGF-1R, and had an inhibitory activity higher than that of the positive control antibody Teprotumumab.

**Table 7 IC₅₀ values of nanoantibody-Fc fusion proteins in competitive binding to IGF-1R and ligands IGF-1 and IGF-2 thereof**

| Name | IC₅₀ (µg/mL) | |
|---|---|---|
| | IGF-1 | IGF-2 |
| 5115-H0-Fc | 0.2365 | 0.1773 |
| 5115-H4-Fc | 0.2271 | 0.1520 |
| 5115-H4-M34-Fc | 0.2694 | 0.2368 |
| 5115-H4-M34-13-Fc | 0.3357 | 0.1889 |
| 5115-H4-M61-Fc | 0.2574 | 0.2439 |
| 5115-H4-M69-Fc | 0.3517 | 0.2999 |
| Teprotumumab | 0.4857 | 0.3985 |

### Example 13 Detection of inhibitory activity of nanobody-Fc fusion proteins on IGF-1-induced proliferation of BaF3 cells overexpressing human IGF-1R

The VHH gene sequences of 5115-H4-M34 and the Fc sequences (IgG1 C220S L234A L235A D356E L358M or IgG1 C220A L234A L235A D356E L358M) were synthesized into full-length sequences. pcDNA3.4 plasmids were digested with NotI/XbaI (NEB, Cat. R0189L, R0145L), and recombined with the above full-length sequences. The recombinant plasmids were transformed into TOP10 competent cells and bacterial solutions were spread and cultured at 37°C for 16-20 h. Single colonies on the plate were picked to conduct colony PCR and electrophoresis to identify positive clones, the positive clones were picked and sequenced, and the clones with correct sequencing were expanded for culture and extracted to obtain plasmids. HEK293 cells were transiently transfected with lipofectamine and incubated on an orbital shaker rotating at 125 rpm in a cell culture incubator at 37°C and 5% CO₂. The supernatant was collected by centrifugation on day 5 and purified by a Protein A affinity chromatography column. The buffer was replaced with a phosphate buffer through dialysis, and nanoantibody-Fc fusion proteins 5115-H4-M34-Fc' and 5115-H4-M34-Fc" were obtained respectively.

BaF3 cells overexpressing human IGF-1R may break away from the dependence of wild-type BaF3 cells on IL-3 and instead rely on the downstream signaling pathway induced by the IGF-1 binding to IGF-1R for growth, and thus may be used as a cellular model to detect the blocking activity of anti-IGF-1R antibodies on the IGF-1 binding to IGF-1R. A BaF3 stably transfected cell line expressing stable human IGF-1R in the logarithmic growth phase was selected, the cells were resuspended in a fresh RPMI1640 culture medium, seeded into a 96-well cell culture plate at 90 µL/well (3,000 cells/well), and placed in a carbon dioxide incubator (37°C, 5% CO₂). A 10× nanobody-Fc fusion protein solution or a control antibody solution was prepared, and added to the 96-well plate seeded with the cells at 10 µL/well, wherein the final protein concentration was 20 µg/mL, with 3.16-fold dilution and 9 concentrations. The final concentration of IGF-1 in the incubation system was 100 ng/mL. After the cell culture plate was further cultured in the carbon dioxide incubator (37°C, 5% CO₂) for 72 h, the cell culture plate was equilibrated to room temperature, and the cell activity was detected using a CellTiter-Glo reagent (Promega, G7573). The cell viability was calculated according to the following formula: Cell viability (%) = (Lum_{test drug} - Lum _{culture medium control})/(Lum _{cell control} - Lum _{culture medium control}) × 100%. The data was analyzed using Graphpad Prism software, and was fit using nonlinear S-curve regression to obtain a dose-effect curve, and IC₅₀ values were calculated therefrom. The fitting results were shown in Table 8. The results showed that both the tested nanoantibody-Fc fusion proteins had a strong inhibitory effect on proliferation of the BaF3 cells overexpressing human IGF-1R, and had an inhibitory effect significantly higher than that of the positive control antibody.

**Table 8 Inhibitory activity of nanobody-Fc fusion proteins on IGF-1-induced proliferation of BaF3 cells overexpressing human IGF-1R**

| Name | IC₅₀(ng/mL) |
|---|---|
| 5115-H4-M34-Fc' | 8.16 |
| 5115-H4-M34-Fc" | 8.51 |
| Teprotumumab | 120.55 |

| | |
|---|---|
| Note: Fc' indicates a subtype of IgG1 C220S L234A L235A; and Fc" indicates a subtype of IgG1 C220A L234A L235A. | |

### Example 14 Detection of inhibitory activity of nanobody-Fc fusion proteins on IGF-1-induced proliferation of MCF-7 cells

Human breast cancer MCF-7 cells in the logarithmic growth phase were digested with trypsin, resuspended in an RPMI1640 culture medium containing 10% FBS, and seeded into a 96-well cell culture plate at a density of 4,000 cells per well. Following culturing for attachment overnight in a carbon dioxide incubator (37°C, 5% CO₂), the culture medium was replaced with 50 µL of a serum-free RPMI1640 culture medium for further culturing in the carbon dioxide incubator for 5 h. A 2× nanobody-Fc fusion protein solution or a control antibody solution was prepared from an RPMI1640 culture medium containing 2% FBS and 20 ng/mL IGF-1, with the highest concentration of 20 µg/mL, 5-fold dilution, and a total of 9 concentrations, and was added to the corresponding wells of the above 96-well plate seeded with the cells at 50 µL/well. The cell culture plate was placed in a carbon dioxide incubator (37°C, 5% CO₂) for 6 d, and the cell viability was detected using a CyQUANT^{®}. The data was analyzed using Graphpad Prism software, and was fit using nonlinear S-curve regression to obtain a dose-effect curve, and IC₅₀ values were calculated therefrom. The fitting results were shown in Table 9. The results showed that both the tested nanoantibody-Fc fusion proteins had a strong inhibitory effect on the IGF-1 induced proliferation of the MCF-7 cells, and had an inhibitory effect significantly higher than that of the positive control antibody Teprotumumab.

**Table 9 Inhibitory activity of nanobody-Fc fusion proteins on IGF-1-induced proliferation of MCF-7 cells**

| Name | IC₅₀(ng/mL) |
|---|---|
| 5115-H4-M34-Fc' | 4.01 |
| 5115-H4-M34-Fc" | 4.89 |
| Teprotumumab | 226.4 |

| | |
|---|---|
| Note: Fc' indicates a subtype of IgG1 C220S L234A L235A D356E L358M; and Fc" indicates a subtype of IgG1 C220A L234A L235A D356E L358M. | |

### Example 15 Detection of inhibitory activity of nanobody-Fc fusion proteins on IGF-1-induced signaling pathways of MCF-7 cells

MCF-7 cells in the logarithmic growth phase were seeded into a 6-well plate (about 4.5*10e5 cells) and cultured overnight in a cell culture incubator at 37°C and 5% CO₂. The culture medium was discarded, the cells were washed with 2 mL of DPBS, a FBS-free fresh cell culture medium was added, and the cells were cultured in a cell culture incubator at 37°C and 5% CO₂ overnight. Nanobody-Fc fusion proteins, positive control antibodies or negative control antibodies (with a final concentration of 2 µg/mL) were added to each well, mixed gently and incubated at 37°C for 10 min. IGF-1 (with a final concentration of 50 ng/mL) was added and incubated at 37°C for 20 min. The culture medium was discarded, the cells were washed with 2 mL of DPBS, and the washing solution was discarded. The cell lysis buffer containing a protease inhibitor was added to each well at 150 µL/well. The cells were lysed on ice for 30 min, and the lysed cells were collected, transferred to a 1.5 mL centrifuge tube, and centrifuged at 4°C and 12,000 rpm for 20 min. The protein concentration of the supernatant was tested by a BCA kit, and the supernatant was added to a 4× loading buffer containing DTT following adjustment of the protein concentration, mixed and heated at 100°C for 5 min. The phosphorylation level of Erk1/2 and the total protein level of Erk1/2 in each sample were detected by Western Blot. The primary antibodies were Phospho-p44/42 MAPK (Erk1/2) (Thr202/Tyr204) (D13.14.4E) XP ^{®} Rabbit mAb (cell signaling, 8544S) and p44/42 MAPK (Erk1/2) (137F5) Rabbit mAb (cell signaling, 4695T), and the secondary antibody was anti-rabbit IgG-HRP second antibody (cell signaling, 7074P2). As shown by the test results in FIG. 3, compared with the blank cell control, IGF-1 stimulation effectively increased the phosphorylation level of Erk1/2, and pretreatment with both the nanobody-Fc fusion proteins 5115-Fc and 5115-H0 could effectively inhibit IGF-1-induced Erk1/2 phosphorylation, and had an inhibitory effect higher than that of the positive control Teprotumumab.

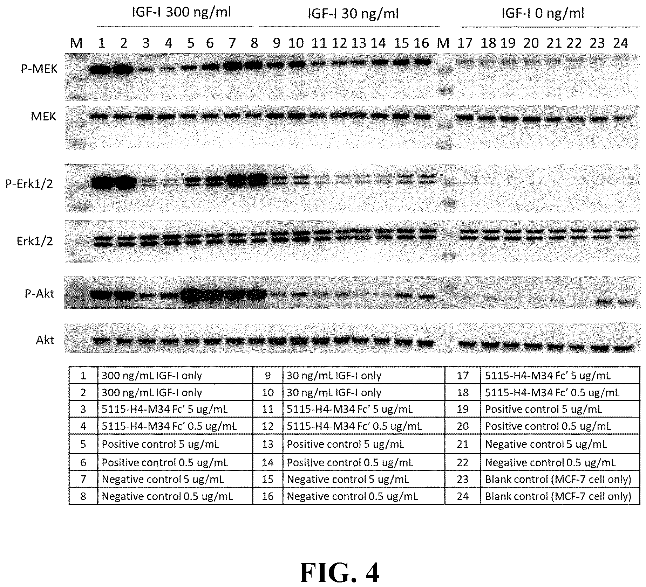

Further, MCF-7 cells in the logarithmic growth phase were seeded into a 6-well plate (about 4.5*10e5 cells) and cultured overnight in a cell culture incubator at 37°C and 5% CO₂. The culture medium was discarded, the cells were washed with 2 mL of DPBS, a FBS-free fresh cell culture medium was added, and the cells were cultured in a cell culture incubator at 37°C and 5% CO₂ overnight. Nanobody-Fc fusion proteins, positive control antibodies or negative control antibodies, with a final concentration of 5 µg/mL or 0.5 µg/mL, were added to each well, mixed gently and incubated at 37°C for 30 min. IGF-1 (with a final concentration of 300 ng/mL or 30 ng/mL) was added and incubated at 37°C for 20 min. The culture medium was discarded, the cells were washed with 2 mL of DPBS, and the washing solution was discarded. The cell lysis buffer containing a protease inhibitor was added to each well at 150 µL/well. The cells were lysed on ice for 30 min, and the lysed cells were collected, transferred to a 1.5 mL centrifuge tube, and centrifuged at 4°C and 12,000 rpm for 20 min. The protein concentration of the supernatant was tested by a BCA kit, and the supernatant was added to a 4× loading buffer containing DTT following adjustment of the protein concentration, mixed and heated at 100°C for 5 min. The phosphorylation level of MEK and the total protein level of MEK, the phosphorylation level of Erk1/2 and the total protein level of Erk1/2, and the phosphorylation level of Akt and the total protein level of Akt in each sample were detected by Western Blot. The primary antibodies were Phospho-p44/42 MAPK (Erk1/2) (Thr202/Tyr204) (D13.14.4E) XP ^{®} Rabbit mAb (CST, #8544), p44/42 MAPK (Erk1/2) (137F5) Rabbit mAb (CST, #4695), Phospho-Akt (Ser473) (D9E) XP ^{®} Rabbit mAb (CST, #4060), Akt (pan) (40D4) Mouse mAb (CST, #2920), Phospho-MEK1/2 (Ser217/221) (41G9) Rabbit mAb (CST, #9154), and MEK1/2 Antibody (CST, 9122); and the secondary antibody was anti-rabbit IgG-HRP second antibody (CST, #7074) or anti-Mouse IgG-HRP (CST, #7076). As shown by the test results in FIG. 4, compared with the blank cell control, IGF-1 stimulation effectively increased the phosphorylation levels of MEK, Erk1/2 and Akt in MCF-7 cells, and as the IGF-1 concentration increased, the protein phosphorylation level increased. The treatment groups had no significant difference in the total protein levels of MEK, Erk1/2 and Akt. Pretreatment with the nanobody-Fc fusion proteins 5115-H4-M34 Fc' at both 5 µg/mL and 0.5 µg/mL could effectively inhibit the IGF-1-induced phosphorylation of MEK, Erk1/2 and Akt, with an inhibitory effect equivalent to that of the positive control on protein phosphorylation under stimulation of 30 ng/mL IGF-1, and an inhibitory effect higher than that of the positive control Teprotumumab on protein phosphorylation under stimulation of 300 ng/mL IGF-1.

## Claims

1. An antigen-binding protein, capable of inhibiting competitive binding of IGF-1R to ligands thereof with an IC₅₀ value of about 1.0 µg/mL or less in a competitive ELISA.

2. The antigen-binding protein according to claim 1, wherein the IGF-1R comprises IGF-1R derived from a primate.

3. The antigen-binding protein according to any one of claims 1-2, wherein the ligands of the IGF-1R comprise IGF-1 and/or IGF-2, and functionally active fragments thereof.

4. An antigen-binding protein, capable of competing with a reference antibody to bind to IGF-1R, wherein the reference antibody comprises HCDR1 to 3, and the HCDR1 to 3 comprise the amino acid sequences set forth in SEQ ID NOs: 85, 100 and 131, respectively.

5. The antigen-binding protein according to claim 4, wherein the reference antibody comprises a variable domain of the heavy chain (VH), and the VH comprises the amino acid sequence set forth in SEQ ID NO: 1.

6. An antigen-binding protein, comprising HCDR3, wherein the HCDR3 comprises the amino acid sequence set forth in SEQ ID NO: 217 or 218.

7. The antigen-binding protein according to any one of claims 1-6, comprising HCDR3, wherein the HCDR3 comprises the amino acid sequence set forth in any one of SEQ ID NOs: 130-153.

8. The antigen-binding protein according to any one of claims 1-7, comprising HCDR2, wherein the HCDR2 comprises the amino acid sequence set forth in SEQ ID NO: 215 or 216.

9. The antigen-binding protein according to any one of claims 1-8, comprising HCDR2, wherein the HCDR2 comprises the amino acid sequence set forth in any one of SEQ ID NOs: 96-129.

10. The antigen-binding protein according to any one of claims 1-9, comprising HCDR1, wherein the HCDR1 comprises the amino acid sequence set forth in SEQ ID NO: 213 or 214.

11. The antigen-binding protein according to any one of claims 1-10, comprising HCDR1, wherein the HCDR1 comprises the amino acid sequence set forth in any one of SEQ ID NOs: 81-95.

12. The antigen-binding protein according to any one of claims 1-11, comprising a variable domain of the heavy chain (VH), wherein the VH comprises HCDR1, HCDR2 and HCDR3; the HCDR3 comprises the amino acid sequence set forth in SEQ ID NO: 217 or 218; the HCDR2 comprises the amino acid sequence set forth in SEQ ID NO: 215 or 216; and the HCDR1 comprises the amino acid sequence set forth in SEQ ID NO: 213 or 214.

13. The antigen-binding protein according to any one of claims 1-12, comprising a variable domain of the heavy chain (VH), wherein the VH comprises the HCDR1, HCDR2 and HCDR3; the HCDR3 comprises the amino acid sequence set forth in any one of SEQ ID NOs: 130-153; the HCDR2 comprises the amino acid sequence set forth in any one of SEQ ID NOs: 96-129; and the HCDR1 comprises the amino acid sequence set forth in any one of SEQ ID NOs: 81-95.

14. The antigen-binding protein according to any one of claims 1-13, comprising HCDR1, HCDR2 and HCDR3, wherein the HCDR1, HCDR2 and HCDR3 comprise the amino acid sequences selected from the following group:
| No. | HCDR1 (SEQ ID NO) | HCDR2 (SEQ ID NO) | HCDR3 (SEQ ID NO) |
|---|---|---|---|
| 1 | 85 | 100 | 131 |
| 2 | 86 | 101 | 133 |
| 3 | 87 | 102 | 131 |
| 4 | 87 | 102 | 131 |
| 5 | 87 | 102 | 131 |
| 6 | 87 | 102 | 131 |
| 7 | 87 | 102 | 131 |
| 8 | 88 | 102 | 131 |
| 9 | 88 | 102 | 131 |
| 10 | 89 | 103 | 131 |
| 11 | 87 | 104 | 134 |
| 12 | 87 | 105 | 134 |
| 13 | 90 | 103 | 134 |
| 14 | 87 | 106 | 134 |
| 15 | 87 | 107 | 135 |
| 16 | 89 | 103 | 134 |
| 17 | 87 | 108 | 135 |
| 18 | 85 | 103 | 136 |
| 19 | 87 | 103 | 134 |
| 20 | 90 | 102 | 135 |
| 21 | 87 | 100 | 134 |
| 22 | 87 | 109 | 137 |
| 23 | 91 | 103 | 134 |
| 24 | 87 | 103 | 134 |
| 25 | 87 | 108 | 131 |
| 26 | 87 | 103 | 138 |
| 27 | 87 | 103 | 134 |
| 28 | 87 | 110 | 131 |
| 29 | 89 | 111 | 139 |
| 30 | 89 | 103 | 139 |
| 31 | 87 | 109 | 135 |
| 32 | 87 | 112 | 140 |
| 33 | 87 | 113 | 138 |
| 34 | 87 | 102 | 141 |
| 35 | 87 | 114 | 131 |
| 36 | 87 | 103 | 135 |
| 37 | 87 | 103 | 131 |
| 38 | 87 | 115 | 134 |
| 39 | 87 | 115 | 135 |
| 40 | 87 | 103 | 134 |
| 41 | 87 | 108 | 142 |
| 42 | 87 | 103 | 134 |
| 43 | 87 | 115 | 143 |
| 44 | 87 | 116 | 131 |
| 45 | 87 | 117 | 131 |
| 46 | 87 | 108 | 138 |
| 47 | 87 | 103 | 137 |
| 48 | 92 | 118 | 134 |
| 49 | 87 | 103 | 144 |
| 50 | 87 | 119 | 145 |
| 51 | 89 | 111 | 146 |
| 52 | 87 | 116 | 147 |
| 53 | 87 | 102 | 135 |
| 54 | 87 | 114 | 131 |
| 55 | 87 | 103 | 148 |
| 56 | 87 | 120 | 131 |
| 57 | 87 | 108 | 131 |
| 58 | 87 | 115 | 148 |
| 59 | 87 | 106 | 135 |
| 60 | 87 | 102 | 135 |
| 61 | 89 | 121 | 134 |
| 62 | 89 | 121 | 138 |
| 63 | 87 | 110 | 149 |
| 64 | 87 | 112 | 135 |
| 65 | 93 | 122 | 134 |
| 66 | 89 | 123 | 146 |
| 67 | 89 | 103 | 150 |
| 68 | 89 | 124 | 134 |
| 69 | 89 | 125 | 134 |
| 70 | 89 | 108 | 146 |
| 71 | 89 | 126 | 151 |
| 72 | 94 | 108 | 134 |
| 73 | 89 | 103 | 134 |
| 74 | 89 | 127 | 152 |
| 75 | 89 | 103 | 134 |
| 76 | 89 | 103 | 146 |
| 77 | 95 | 128 | 153 |
| 78 | 89 | 129 | 134 |
| 79 | 95 | 103 | 134 |
| 80 | 85 | 124 | 134 |

15. The antigen-binding protein according to any one of claims 1-14, comprising H-FR1, wherein the C-terminus of the H-FR1 is directly or indirectly linked to the N-terminus of the HCDR1, and the H-FR1 comprises the amino acid sequence set forth in any one of SEQ ID NOs: 219, 220, and 154-186.

16. The antigen-binding protein according to any one of claims 1-15, comprising H-FR2, wherein the H-FR2 is located between the HCDR1 and the HCDR2, and the H-FR2 comprises the amino acid sequence set forth in any one of SEQ ID NOs: 221, 222, and 187-195.

17. The antigen-binding protein according to any one of claims 1-16, comprising H-FR3, wherein the H-FR3 is located between the HCDR2 and the HCDR3, and the H-FR3 comprises the amino acid sequence set forth in any one of SEQ ID NOs: 223 and 196-203.

18. The antigen-binding protein according to any one of claims 1-17, comprising H-FR4, wherein the N-terminus of the H-FR4 is directly or indirectly linked to the C-terminus of the HCDR3, and the H-FR4 comprises the amino acid sequence set forth in any one of SEQ ID NOs: 224 and 204-206.

19. The antigen-binding protein according to any one of claims 1-18, comprising H-FR1, H-FR2, H-FR3 and H-FR4, wherein the H-FR1 comprises the amino acid sequence set forth in SEQ ID NO: 219 or 220; the H-FR2 comprises the amino acid sequence set forth in SEQ ID NO: 221 or 222; the H-FR3 comprises the amino acid sequence set forth in SEQ ID NO: 223; and the H-FR4 comprises the amino acid sequence set forth in SEQ ID NO: 224.

20. The antigen-binding protein according to any one of claims 1-19, comprising a variable domain of the heavy chain (VH), wherein the VH comprises the amino acid sequence set forth in SEQ ID NO: 225 or 226.

21. The antigen-binding protein according to any one of claims 1-20, comprising a variable domain of the heavy chain (VH), wherein the VH comprises the amino acid sequence set forth in any one of SEQ ID NOs: 1-80.

22. The antigen-binding protein according to any one of claims 1-21, comprising an antibody or an antigen-binding fragment thereof.

23. The antigen-binding protein according to any one of claims 1-22, comprising a single domain antibody or an antigen-binding fragment thereof.

24. The antigen-binding protein according to claim 22, wherein the antigen-binding fragment is selected from the group consisting of Fab, Fab', F(ab)2, Fv fragment, F(ab')2, scFv, di-scFv, VHH and/or dAb.

25. The antigen-binding protein according to claim 22, wherein the antibody comprises an alpaca antibody, a chimeric antibody, a humanized antibody, and/or a fully human antibody.

26. The antigen-binding protein according to any one of claims 1-25, comprising the amino acid sequence set forth in any one of SEQ ID NOs: 1-80.

27. A polypeptide, comprising the antigen-binding protein according to any one of claims 1-26.

28. The polypeptide according to claim 27, further comprising a structure that improves the stability of the antigen-binding protein and/or is capable of binding to an Fc receptor.

29. The polypeptide according to any one of claims 27-28, further comprising an immunoglobulin Fc region.

30. The polypeptide according to claim 29, wherein the antigen-binding protein is directly or indirectly linked to the Fc region.

31. The polypeptide according to any one of claims 29-30, wherein the C-terminus of the VH of the antigen-binding protein is directly or indirectly linked to the N-terminus of the Fc region.

32. The polypeptide according to any one of claims 29-31, wherein the Fc region comprises Fc derived from IgG1 and/or Fc derived from IgG4.

33. The polypeptide according to any one of claims 29-32, wherein the Fc region comprises the amino acid sequence set forth in SEQ ID NO: 227.

34. The polypeptide according to any one of claims 29-33, wherein the Fc region comprises the amino acid sequence set forth in any one of SEQ ID NOs: 209-212.

35. An immunoconjugate, comprising the isolated antigen-binding protein according to any one of claims 1-26 and/or the polypeptide according to any one of claims 27-34.

36. A nucleic acid, encoding the isolated antigen-binding protein according to any one of claims 1-26 and/or the polypeptide according to any one of claims 27-34.

37. A vector, comprising the nucleic acid according to claim 36.

38. A cell, comprising the isolated antigen-binding protein according to any one of claims 1-26, the polypeptide according to any one of claims 27-34, the immunoconjugate according to claim 35, the nucleic acid according to claim 36, and/or the vector according to claim 37.

39. A method for preparing the isolated antigen-binding protein according to any one of claims 1-26 and/or the polypeptide according to any one of claims 27-34, wherein the method comprises culturing the cell according to claim 38 under the conditions of allowing expression of the isolated antigen-binding protein and/or the polypeptide.

40. A composition, comprising the isolated antigen-binding protein according to any one of claims 1-26, the polypeptide according to any one of claims 27-34, the immunoconjugate according to claim 35, the nucleic acid according to claim 36, the vector according to claim 37, and/or the cell according to claim 38, and optionally a pharmaceutically acceptable adjuvant.

41. A kit, comprising the isolated antigen-binding protein according to any one of claims 1-26, the polypeptide according to any one of claims 27-34, the immunoconjugate according to claim 35, the nucleic acid according to claim 36, the vector according to claim 37, the cell according to claim 38, and/or the composition according to claim 40.

42. A method for inhibiting proliferation of cells overexpressing IGF-1R or a functionally active fragment thereof, inhibiting an MAPK pathway and/or inhibiting interaction between IGF-1R or a functionally active fragment thereof and ligands thereof, comprising administering the isolated antigen-binding protein according to any one of claims 1-26, the polypeptide according to any one of claims 27-34, the immunoconjugate according to claim 35, the nucleic acid according to claim 36, the vector according to claim 37, the cell according to claim 38, the composition according to claim 40, and/or the kit according to claim 41.

43. The method according to claim 42, wherein the inhibiting an MAPK pathway comprises inhibiting phosphorylation of an MAPK pathway protein.

44. The method according to any one of claims 42-43, wherein the MAPK pathway protein comprises MEK, Erk1/2 and Akt, or functionally active fragments thereof.

45. The method according to any one of claims 42-44, wherein the ligands of the IGF-1R comprise IGF-1 and/or IGF-2, and functionally active fragments thereof.

46. A method for detecting the presence and/or content of IGF-1R or a functionally active fragment thereof, comprising administering the isolated antigen-binding protein according to any one of claims 1-26, the polypeptide according to any one of claims 27-34, the immunoconjugate according to claim 35, the nucleic acid according to claim 36, the vector according to claim 37, the cell according to claim 38, the composition according to claim 40, and/or the kit according to claim 41.

47. Use of the isolated antigen-binding protein according to any one of claims 1-26, the polypeptide according to any one of claims 27-34, the immunoconjugate according to claim 35, the nucleic acid according to claim 36, the vector according to claim 37, the cell according to claim 38, the composition according to claim 40, and/or the kit according to claim 41 in preparation of a drug for preventing, alleviating, and/or treating a disease or disorder.

48. The use according to claim 47, wherein the disease or disorder comprises a tumor.

49. The use according to any one of claims 47-48, wherein the disease or disorder comprises a tumor associated with expression of IGF-1R or a functionally active fragment thereof.

50. The use according to any one of claims 47-49, wherein the disease or disorder comprises breast cancer.
